# EUROPEAN PATENT SPECIFICATION

(11) **EP 4 436 611 B1**
(45) Date of publication and mention of the grant of the patent: **18.03.2026**
(21) Application number: 22823332.6
(22) Date of filing: 24.11.2022
(51) Int. Cl.: A61K 47/64, C08G 69/10, C08G 69/36, C08G 69/48, C12N 15/88

(54) **COMPLEXES COMPRISING A SHIELDING COMPONENT**
KOMPLEXE MIT EINER ABSCHIRMKOMPONENTE
COMPLEXES COMPRENANT UN COMPOSANT DE PROTECTION

(30) Priority: 25.11.2021 EP 21383069
(43) Date of publication of application: 02.10.2024
(73) Proprietor: Polypeptide Therapeutic Solutions, S.L., 46980 Paterna (ES); The Regents of the University of California, Los Angeles, CA 90095-7191 (US)
(72) Inventor: DEMING, Timothy Jon, LOS ANGELES, California 90049 (US); LEON, Carles Felip, 46980 PATERNA (ES); DOLZ PÉREZ, Irene, 46980 PATERNA (ES); ESTEBAN PÉREZ, Sergio, 46980 PATERNA (ES); HERRERA MUÑOZ, Lidia, 46980 PATERNA (ES); NEBOT CARDA, Vicent Josep, 46980 PATERNA (ES)
(74) Representative: ZBM Patents - Zea, Barlocci & Markvardsen
(86) International application number: PCT/EP2022/083118
(87) International publication number: WO 2023/094518

(56) References cited:
- WO-A1-2019/067676
- WANG C ET AL: "Poly(alpha-glutamic acid) combined with polycation as serum-resistant carriers for gene delivery", INTERNATIONAL JOURNAL OF PHARMACEUTICS, ELSEVIER, NL, vol. 398, no. 1-2, 15 October 2010 (2010-10-15), pages 237 - 245, XP027261272, ISSN: 0378-5173, [retrieved on 20100803]
- TIAN HUAYU ET AL: "pH-responsive zwitterionic copolypeptides as charge conversional shielding system for gene carriers", JOURNAL OF CONTROLLED RELEASE, ELSEVIER, AMSTERDAM, NL, vol. 174, 14 November 2013 (2013-11-14), pages 117 - 125, XP028810743, ISSN: 0168-3659, DOI: 10.1016/J.JCONREL.2013.11.008
- YINTAO SUN ET AL: "Conformation-Directed Formation of Self-Healing Diblock Copolypeptide Hydrogels via Polyion Complexation", JOURNAL OF THE AMERICAN CHEMICAL SOCIETY, vol. 139, no. 42, 12 October 2017 (2017-10-12), pages 15114 - 15121, XP055586636, ISSN: 0002-7863, DOI: 10.1021/jacs.7b08190

## Description

This application claims the benefit of European Patent Application EP21383069.8 filed on November 25, 2021.

### Technical Field

.. The present disclosure relates to new polymer complexes comprising a shielding component which is an anionic copolymer comprising a methionine sulfoxide-based block. These polymer complexes may be used as non-viral vectors for delivery of active ingredients, including nucleic acids, to cells.

### Background Art

.. Use of cationic polymers as non-viral synthetic carriers for delivering active ingredients, and more particularly of nucleic acids, to a target cell have attracted considerable attention. Thus, the polyion complex (PIC) specifically formed by electrostatic interaction between a nucleic acid acting as a polyanion and a cationic polymer (polycation) received the name of polyplex and have been disclosed extensively in the prior art. However, applicability and translation from lab bench to commercialization remains a challenge.

.. One major reason for limited development of polycation-based non-viral vectors resides in that it is required that the cationic polymer show different functions at different stages of the delivery process. For example, it may be necessary for the polymeric carrier to have a high amine density and appropriate pKa to overcome the endosomal membrane barrier since a protonated potential of the nanoparticle could be a cause of endosome buffering and membrane destabilization. On the contrary, the positively-charged property of the nanoparticle may cause aggregation in the blood stream and non-specific interaction with a negatively-charged serum components, thereby producing a thrombus in the blood capillary. This has a risk of disturbing construction of a plasma membrane and a risk of inducing high cytotoxicity and excess immune response. Additionally, these positively charged nanoparticles may cause severe serum inhibition and are rapidly cleared from the blood, which will hinder their applications in vivo.

.. A well-known attempt to solve these problems is the introduction of a neutral charge shielding by covering the surface of the nanoparticle with polyethylene glycol (PEG). Polyethylene glycol (PEG) is widely used in drug delivery and nanotechnology because of its stealth properties and biocompatibility. Thanks to these properties particulate delivery systems containing PEG are able to avoid aggregation, evade the immune system, and, as a result, have a prolonged circulation time within the body.

.. Thus, it is also well known that polycations having a polyethylene glycol (PEG) portion, result in a polyplex having a structure wherein the nucleic acid is condensed as a result of the interaction of the nucleic acid with the polycation portion in the block copolymer to form a core portion, and wherein the hydrophilic and bio-compatible PEG portion in the block copolymer forms a shell surrounding the core portion. Thus, the PIC is able to stably encapsulate a nucleic acid, and it is able to avoid the foreign substance-recognizing mechanism existing in vivo.

.. However, the presence of covalently bonded PEG will significantly reduce the transfection efficiency in case of the polyplexes because the neutral surface of the nanoparticles can decrease the cellular uptake efficiency or may also cause activity loss in case of proteins because the hindered space of the active site. It is also well known that PEG generates anti-PEG antibodies that may cause inmunogenicity, allergic reactions and anaphylactic shock.

Wang et al (Int J Pharm. 2010;398(1-2):237-45) and Tian et al (J Control. Release. 2014;174:117-125) disclose a polyplex shielded by an anionic polymer.

.. Further, polyion complex polypeptide hydrogels comprising at least two diblock, triblock or pentablock copolypeptides and water have been disclosed. Thus, e.g. WO2019067676 discloses hydrogels wherein each copolypeptide has an ionic segment with the opposite charge from the other, and wherein both copolypeptides comprise methionine sulfoxide (M°) segments. In particular, WO2019067676 describes a PIC hydrogels formed by (M^{o}A)ₙKₓ, (M^{o}A)ₙEₓ and water.

.. Hydrogel systems have potential applications as a bioink for tissue regeneration and injectable depot/carrier for small molcule or protein controlled release applications. Gels are a colloidal state of matter in which a small amount of a solid-like microphase-separated component network (gelator) is able to immobilize the bulk flow of a larger amount of liquid-like phase. This microphase separation is induced by nucleation. As a result, the material is "solid-like" in its rheological behaviour: the storage (elastic) modulus (G') is larger than the loss (viscous) modulus (G") over a shear frequency range within the linear viscoelastic region.

.. Therefore, from what it is known in the field, there is still the need to develop alternative polycation-based non-viral vectors which overcome the disadvantages of the prior art.

### Summary of Invention

.. The inventors have found that some anionic copolymers comprising a methionine sulfoxide-based blocks are useful as shielding for positively charged proteins or polycation-based non-viral vectors for delivery of proteins or active ingredients, including nucleic acids, to cells with low cytotoxicity, high efficiency, potential adequate plasma half-life time, potential enhanced permeability and retention, high solubility in aqueous solution, high stability due to limited or even completely suppressed aggregation issues in the bloodstream, and potential different cell and tissue tropism.

.. It is an object of the present invention to provide new polycation-based non-viral vectors, i.e. polymer complexes, comprising a cationic polymer, at least one pharmaceutically, veterinary or cosmetically active ingredient, and a shielding component which is an anionic copolymer comprising a methionine sulfoxide-based block.

.. Thus, according to one aspect of the present invention, it is provided a polymer complex comprising:
a) a positively charged nanoparticle comprising a cationic polymer covalently or electrostatically bonded with at least one pharmaceutically, veterinary or cosmetically active ingredient; and
b) at least one anionic copolymer selected from
   i. a copolymer comprising substructure I;
   ii. a copolymer comprising substructure II; and
   iii. a copolymer comprising substructure III;

wherein the substructures I II, and III are depicted as follows:

   -Aₘ-Bₙ- Substructure I

   -(Bₙ-Aₘ)ₚ-Bₙ- Substructure II

   -(Aₘ-Bₙ)ₚ-Aₘ- Substructure III
each instance of A is an amino acid residue independently selected from methionine sulfoxide, ethionine sulfoxide, S-alkyl-cysteine sulfoxide, S-alkyl cysteine sulfone, S-alkyl homocysteine, S-alkyl homocysteine sulfoxide, glycosylated cysteine, serine, homoserine, homomethionine sulfoxide, sarcosine, glycine, and alanine;
wherein at least 50 mol% of the A amino acid residues are methionine sulfoxide;
wherein each instance of B is independently selected from glutamic acid, aspartic acid and a salt thereof;
m is an integer from 10 to 600;
n is an integer from 5 to 200;
p is an integer from 1 to 2.

.. It is also an object of the present invention to provide new protein-based complexes comprising a cationically charged protein, and a shielding component which is an anionic copolymer comprising of a methionine sulfoxide-based block.

.. Thus, according to an aspect of the present invention, it is provided a protein-based complex comprising
a) a cationically charged protein; and
b) at least one anionic copolymer selected from
   i. a copolymer comprising substructure I;
   ii. a copolymer comprising substructure II; and
   iii. a copolymer comprising substructure III;
wherein the substructures I, II, and III are as defined herein.

.. In accordance with another aspect of the present invention, it is provided the use of at least one anionic copolymer selected from
i. a copolymer comprising substructure I;
ii. a copolymer comprising substructure II; and
iii. a copolymer comprising substructure III;

as shielding for positively charged proteins or positively charged nanoparticles comprising a cationic polymer covalently or electrostatically bonded with at least one pharmaceutically, veterinary or cosmetically active ingredient, for delivery of proteins or pharmaceutically, veterinary or cosmetically active ingredients to a biological target;
wherein substructures I, II, and III are as defined herein.

.. An additional aspect of the present invention relates to a pharmaceutical, veterinary or cosmetical composition comprising at least one polymer complex or at least one protein based complex as defined herein, together with one or more appropriate acceptable excipients.

.. In a further aspect of the present invention, it is provided a polymer complex of the invention for use in a method of delivering at least one pharmaceutically, veterinary or cosmetically active ingredient to a biological target, the method comprising:
a) providing the polymer complex, the protein-based complex or the compositions as described herein; and
b) contacting the biological target with the polymer complex, the protein-based complex or the composition.

.. This aspect may also be referred as polymer complex for use in a method for delivering at least one pharmaceutically, veterinary or cosmetically active ingredient to a target cell, which comprises: administering a solution that contains the polymer complex, the protein-based complex or the composition as defined herein to an animal, including human, so that the polymer complex, the protein-based complex or the composition may be introduced into the target cell; transferring the polymer complex, the protein-based complex or the composition from the endosome to the cytoplasm; dissociating the polymer complex, the protein-based complex or the composition in the cell; and releasing the active ingredient into the cytoplasm.

.. In a further aspect of the present invention, it is provided a method for the preparation of a polymer complex which comprises
a) providing a cationic polymer in a first liquid;
b) providing at least one pharmaceutically, veterinary or cosmetically active ingredient in a second liquid;
c) contacting the cationic polymer in the first liquid with the at least one pharmaceutically, veterinary or cosmetically active ingredient in the second liquid to form a positively charged nanoparticle;
d) providing an anionic copolymer selected from
   i. a copolymer comprising substructure I;
   ii. a copolymer comprising substructure II; and
   iii. a copolymer comprising substructure III;
e) contacting the positively charged nanoparticle with the anionic copolymer to form a polymer complex;
wherein the substructures I II, and III are as defined herein.

.. Alternatively, it is provided a method for the preparation of a polymer complex which comprises
a) providing a cationic polymer in a first liquid;
b) providing at least one pharmaceutically, veterinary or cosmetically active ingredient in a second liquid and mixing it with an anionic copolymer selected from
   i. a copolymer comprising substructure I;
   ii. a copolymer comprising substructure II; and
   iii. a copolymer comprising substructure III;
c) contacting the cationic polymer in the first liquid with the at least one pharmaceutically, veterinary or cosmetically active ingredient and the anionic copolymer in the second liquid to form a shielded nanoparticle;
wherein the substructures I, II, and III are as defined herein.

.. In one aspect of the present invention, it is provided a polymer complex, a protein-based complex, or a composition as defined herein, for use as a medicament.

.. In one aspect of the present invention, it is provided a polymer complex, the protein-based complex, or a composition as defined herein, for use (i) as transfection reagent for transfecting at least one active agent into a cell; (ii) for use in the in vivo or ex vivo therapies encoding a recombinant protein, a peptide or an antibody; iii) in the production of peptides, proteins, antibodies, or recombinant virus; (iv) for use as a therapeutic or prophylactic vaccine against viral infections or as a therapeutic vaccine against cancers; and (v) for use in genome engineering, for cell reprogramming, for differentiating cells or for gene-editing.

.. In another aspect of the present invention, it is provided a protein-based complex or a composition containing it, for use in protein-based therapy.

.. In a further aspect of the present invention, it is provided a device for delivering at least one pharmaceutically, veterinary or cosmetically active ingredient into a cell, comprising the polymer complex or the composition containing it.

### Brief Description of Drawings

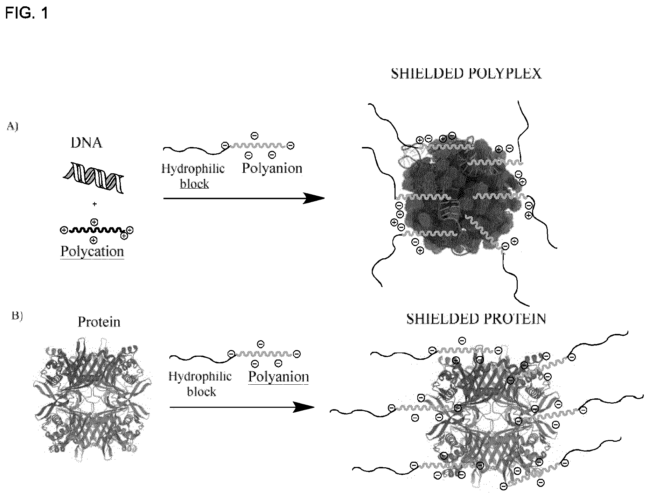

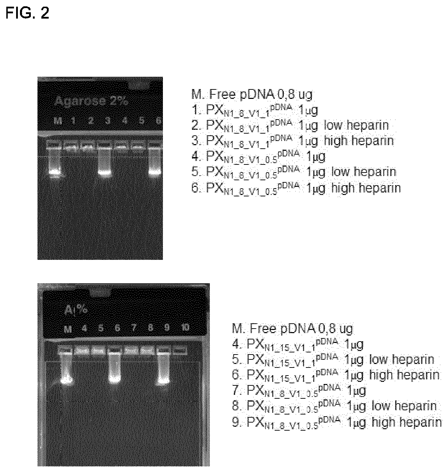

.. Non-limiting examples of the present disclosure will be described in the following, with reference to the appended drawings, in which
.. Figure 1 shows a schematic representation of the formation of the shielded complexes: A) Tricomponent complex formed by genetic material, polycation and a shielding block anionic copolymer. B) Assembly of a protein and a shielding block polyanionic copolymer.
.. FIG. 2 shows the result of the polyplexes PX_{N1_8_V1_1}^{pDNA} and PX_{N1_8_V1_0.5}^{pDNA} (top), and PX_{N1_15_V1_1}^{pDNA} and PX_{N1_15_V1_0.5}^{pDNA} (bottom) with shielding polymer V1 analyzed by agarose gel electrophoresis technique. This technique shows, in a qualitative manner, the ability of complexation of the polyplexes towards the genetic material (DNA). Also, it shows the ability to release the genetic material in the presence of low and high concentration of a polyanionic competing agent (heparin) at low and high concentrations. In the lane labeled as M, the free pDNA is seeded. As can be seen, the free genetic material is shining under the UV transiluminator. In lanes 1 and 4 (top drawing) and 4 and 7 (bottom drawing), the polyplex is seeded and it can be seen that when the polycation is present and the polyplex is formed, the genetic material is entrapped, and no signal can be observed. In lanes 2 and 5 (top drawing) and 5 and 8 (bottom drawing), the polyplex is seeded in presence of low concentration of heparin competitor, showing no release in those conditions. In lanes 3 and 6 (top drawing) and 6 and 9 (bottom drawing), the polyplex is seeded together with high concentration of anionic heparin competitor, in this case, the release of the genetic material is observed. This behavior is the ideal one because the polyplexes need to be stable at low concentrations of competing molecules extracellularly but should be labile enough to release the cargo when an intracellular stimulus is applied.

### Detailed description of the invention

.. All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

.. As used herein, the indefinite articles "a" and "an" are synonymous with "at least one" or "one or more." Unless indicated otherwise, definite articles used herein, such as "the" also include the plural of the noun.

.. The term "substituted" means that one or more hydrogen atoms on the designated atom or group are replaced with a selection from the indicated group, provided that the designated atom's normal valency under the existing circumstances is not exceeded. Combinations of substituents and/or variables are permissible. The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen atom. It is possible that groups in the compounds according to the invention are substituted with one, two, three, four or five identical or different substituents, particularly with one, two or three substituents.

.. As used herein, the phrase "natural amino acid" refers to the any of the 20 amino acids naturally occurring in proteins. Such natural amino acids include the nonpolar, or hydrophobic amino acids, glycine, alanine, valine, leucine isoleucine, methionine, phenylalanine, tryptophan, and proline. Cysteine is sometimes classified as nonpolar or hydrophobic and other times as polar. Natural amino acids also include polar, or hydrophilic amino acids, such as tyrosine, serine, threonine, aspartic acid (also known as aspartate, when charged), glutamic acid (also known as glutamate, when charged), asparagine, and glutamine. Certain polar, or hydrophilic, amino acids have charged side-chains, depending on environmental pH. Such charged amino acids include lysine, arginine, and histidine. One of ordinary skill in the art would recognize that protection of a polar or hydrophilic amino acid side-chain can render that amino acid nonpolar. For example, a suitably protected tyrosine hydroxyl group can render that tyrosine nonpolar and hydrophobic by virtue of protecting the hydroxyl group.

.. As used herein, the phrase "unnatural amino" refers to amino acids not included in the list of 20 amino acids naturally occurring in proteins, as described above. Such amino acids include the D-isomer of any of the 19 naturally occurring amino acids, glycine is achiral. Unnatural amino acids also include homoserine and ornithine. Other unnatural amino acids side-chains are well known to one of ordinary skill in the art and include unnatural aliphatic side chains. Other unnatural amino acids include modified amino acids, including those that are N-alkylated, cyclized, phosphorylated, acetylated, amidated, azidylated, labelled, and the like.

.. As used herein, the term "repeating unit", or "block" refers to a repeating monomeric unit. A repeating unit or a block may consist of a single monomer or may be comprised of one or more monomers, resulting in a "mixed block".

.. One skilled in the art will recognize that a monomer repeating unit is defined by square brackets ("[ ]") depicted around the repeating monomer unit. The number (or letter representing a numerical range) on the lower right of the brackets represents the number of monomer units that are present in the polymer chain.

.. In accordance with some embodiments, optionally in combination with any of the embodiments provided above or below, at least one anionic copolymer comprising the substructure I, II, or III interacts electrostatically with positively charged proteins, acting as a shielding layer on top of the protein, or intercalated among it.

.. It is noted that, in view of the nomenclature used herein for the shielding polymers according to the invention, the numerical values mentioned in parenthesis refer to the degree of polymerization (DP) for each monomeric unit as a statistical number. The DP for a particular monomer unit is calculated dividing the molecular weight of the polymer by the molecular weight of the monomer unit. The DP value is subject to a reasonable uncertainty, due to the ring-opening polymerization mechanism, which, in the context of the present invention, may be considered within the range ±20%, preferably ±15%, more preferably ±10%, even more preferably ±5%, being particularly preferred ±2%.

.. That at least one anionic copolymer is selected from a copolymer comprising substructure I; a copolymer comprising substructure II; and a copolymer comprising substructure III; wherein the substructures I II, and III are depicted above.

.. In the copolymers of substructure I, II, and III, m is an integer from 10 to 600. In one embodiment, optionally in combination with any of the embodiments provided above or below, m is an integer from 10 to 400; more particularly from 15 to 300, even more particularly from 20 to 200, and even more particularly from 25 to 180. Other particular ranges are from 40 to 380, from 50 to 350, from 75 to 300, from 90 to 250, and from 100 to 200. The m integer corresponds to the degree of polymerization of the A amino acid residue, which may be measured by SEC or 1H-NMR spectroscopy as indicated in the examples.

.. In the copolymers of substructure I, II, and III, n is an integer from 5 to 200. In one embodiment, optionally in combination with any of the embodiments provided above or below, n is an integer from 8 to 150; more particularly from 10 to 120, even more particularly from 10 to 110, and even more particularly from 10 to 100. Other particular ranges are from 5 to 190, from 6 to 180, from 7 to 170, from 12 to 120, and from 15 to 80. The n integer corresponds to the degree of polymerization of the B amino acid residue, which may be measured by SEC or 1H-NMR spectroscopy as indicated in the examples.

.. In a particular embodiment, optionally in combination with any of the embodiments provided above or below, in the copolymers of substructure I, II, and III as defined herein the ratio between m:n ranges from 1:1 to 120:1; preferably from 2:1 to 60:1, more preferably from 4:1 to 20:1 and even more preferably from 5:1 to 10:1

.. In the copolymers of substructure I, II, and III, each instance of A is an amino acid residue independently selected from methionine sulfoxide, ethionine sulfoxide, S-alkyl-cysteine sulfoxide, S-alkyl cysteine sulfone, S-alkyl homocysteine, S-alkyl homocysteine sulfoxide, glycosylated cysteine, serine, homoserine, homomethionine sulfoxide, sarcosine, glycine, and alanine; wherein at least 50 mol% of the A amino acid residues are methionine sulfoxide.

.. In one embodiment, optionally in combination with any of the embodiments provided above or below, for each instance of A, 100 mol% of the A amino acid residues are methionine sulfoxide.

.. In one embodiment, optionally in combination with any of the embodiments provided above or below, for each instance of A, at least 50 mol% of the A amino acid residues are methionine sulfoxide; more particularly from 60 mol% to 98 mol% of the A amino acid residues are methionine sulfoxide; even more particularly from 75 mol% to 95 mol%, and even more particularly from 80 mol% to 90 mol%.

.. In one embodiment, optionally in combination with any of the embodiments provided above or below, for each instance of A, from 50 mol% to 98 mol% of the A amino acid residues are methionine sulfoxide, and the remaining A amino acid residues are independently selected from sarcosine, glycine and alanine; more particularly from 60 mol% to 98 mol% of the A amino acid residues are methionine sulfoxide, and the remaining A amino acid residues are independently selected from sarcosine, glycine and alanine; even more particularly from 75 mol% to 95 mol% of the A amino acid residues are methionine sulfoxide, and the remaining A amino acid residues are independently selected from sarcosine, glycine and alanine.

.. In one embodiment, optionally in combination with any of the embodiments provided above or below, each instance of B is glutamic acid.

.. In one embodiment, optionally in combination with any of the embodiments provided above or below, the at least one anionic copolymer comprises a backbone selected from
(1) Poly((L-methionine sulfoxide)138-stat-(L-alanine)19-block-poly(L-glutamate)65, (M^{O})138A19E65;
(2) Poly((L-methionine sulfoxide)167-stat-(L-alanine)25-block-poly(L-glutamate)82, (M^{O})167A25E82;
(3) Poly((L-methionine sulfoxide)154-stat-(L-alanine)25-block-poly(L-glutamate)84, (M^{O})154A25E84;
(4) Poly((L-methionine sulfoxide)139-stat-(L-alanine)20-block-poly(L-glutamate)98, (M^{O})139A20E98;
(5) Poly((L-methionine sulfoxide)167-stat-(L-alanine)25-block-poly(L-glutamate)80, (M^{O})167A25E80;
(6) poly(L-methionine sulfoxide 0.88-stat-L-alanine 0.12)155-block-poly(L-glutamate)30, (M^{O}A)155E30;
(7) poly(L-methionine sulfoxide 0.88-stat-L-alanine 0.12)155-block-poly(L-glutamate)60, (M^{O}A)155E60;
(8) poly(L-methionine sulfoxide 0.88-stat-L-alanine 0.12)155-block-poly(L-glutamate)65, (M^{O}A)155E65;
(9) poly(L-methionine sulfoxide 0.88-stat-L-alanine 0.12)155-block-poly(L-glutamate)90, (M^{O}A)155E90;
(10) poly(L-methionine sulfoxide 0.88-stat-L-alanine 0.12)155-block-poly(L-glutamate)120, (M^{O}A)155E120;
(11) poly(L-methionine sulfoxide 0.88-stat-L-alanine 0.12)100-block-poly(L-glutamate)30, (M^{O}A)100E30;
(12) poly(L-methionine sulfoxide)155-block-poly(L-glutamate)60, (M^{O})155E60;
(13) poly(L-methionine sulfoxide)60-block- poly(L-glutamate)10, (M^{O})60E10;
(14) poly(L-methionine sulfoxide)60-block- poly(L-glutamate)20, (M^{O})60E20;
(15) poly(L-methionine sulfoxide)60-block- poly(L-glutamate)30, (M^{O})60E30;
(16) poly(L-methionine sulfoxide)60-block- poly(L-glutamate)40, (M^{O})60E40;
(17) poly(L-methionine sulfoxide)60-block- poly(L-glutamate)60, (M^{O})60E60;
(18) poly(L-methionine sulfoxide)60-block-(racemic)poly(L-glutamate)60, (M^{O})60(racemic-E)60;
(19) poly(L-methionine sulfoxide 0.88-stat-L-alanine 0.12)50-block-poly(L-glutamate)30-block- poly(L-methionine sulfoxide 0.88-stat-L-alanine 0.12)50, (M^{O}A)50E30(M^{O}A)50;
(20) poly(L-methionine sulfoxide 0.88-stat-L-alanine 0.12)50-block-poly(L-glutamate)30-block- poly(L-methionine sulfoxide 0.88-stat-L-alanine 0.12)100-block-poly(L-glutamate)30-block-poly(L-methionine sulfoxide 0.88-stat-L-alanine 0.12)50, (M^{O}A)50E30(M^{O}A)100E30(M^{O}A)50;
(21) poly(L-glutamate)30-block-poly(L-methionine sulfoxide 0.88-stat-L-alanine 0.12)100-block-poly(L-glutamate)30, E30(MOA)100E30.

.. The anionic copolymers comprising substructure I, II, and III are polymeric structures containing a methionine sulfoxide-based moiety as a repeating unit. These polymers are characterized by a molecular weight (which may be average molecular weight (MW) or number average molecular weight (Mn)), a degree of polymerization, and a polydispersity index. The molecular weight may be measured by methods well-known in the art such as size exclusion chromatography (SEC) (also referred to as gel permeation chromatography (GPC)) matrix assisted laser desorption ionization-time-of-flight mass spectrometry (MALDI-TOF MS) or 1H-NMR spectroscopy, for example. Some of these methods are indicated in more detail in the examples below.

.. The term "polydispersity index" (PDI) is used as a measure of broadness of molecular weight distribution. The larger the PDI, the broader the molecular weight. PDI of a polymer is calculated as the ratio of weight average (MW) by number average (Mn) molecular weight:

.. According to another embodiment, optionally in combination with one or more features of the various embodiments described above or below, the average molecular weight (Mw) of the anionic copolymers comprising substructure I, II, and III is from 2000 to 50000 Da, more particularly from 5000 to 40000 Da, and even more particularly from 10000 to 35000 Da or from 20000 to 30000 Da, measured by SEC as disclosed in the examples.

.. In another embodiment, optionally in combination with one or more features of the various embodiments described above or below, the polydispersity index of the anionic copolymers comprising substructure I, II, and III is from 1.01 to 2.00, or from 1.01 to 1.8, or from 1.01 to 1.50, or from 1.01 to 1.30, or from 1.01 to 1.25 measured by SEC as disclosed in the examples.

.. The polymer complex of the present invention may be a nanoparticle, wherein an active ingredient, preferably a nucleic acid, interacts with a cationic polymer forming a positively charged nanoparticle to form a core portion, and wherein the anionic copolymer forms a shell portion around the aforementioned core portion (see FIG 1A).

.. The positively charged nanoparticle may be easily prepared by mixing an active ingredient with a cationic polymer in any given buffer, for example. The conditions for preparation, such as the aqueous medium, pH, temperature and ionic strength may be appropriately adjusted by those skilled in the art.

.. The term "nanoparticle" as used herein, refers to a particle with at least two dimensions at the nanoscale, particularly with all three dimensions at the nanoscale. Particularly, when the nanoparticle is substantially rod-shaped with a substantially circular cross-section, such as a nanowire or a nanotube, the "nanoparticle" refers to a particle with at least two dimensions at the nanoscale, this two dimensions being the cross-section of the nanoparticle.

.. As used herein, the term "size" refers to a characteristic physical dimension. For example, in the case of a nanoparticle that is substantially spherical, the size of the nanoparticle corresponds to the diameter of the nanoparticle. In the case of a nanoparticle that is substantially rod-shaped with a substantially circular cross-section, such as as nanowire or a nanotube, the size of the nanoparticle corresponds to the diameter of the cross-section of the nanoparticle. In the case of a nanoparticle that is substantially box-shaped, such as a nanocube, a nanobox, or a nanocage, the size of the nanoparticle corresponds to the maximum edge length. When referring to a set of nanoparticles as being of a particular size, it is contemplated that the set of nanoparticles can have a distribution of sizes around the specified size. Thus, as used herein, a size of a set of nanoparticles can refer to a mode of a distribution of sizes, such as a peak size of the distribution of sizes.

.. The anionic copolymers comprising substructure I, II, and III may exist as geometric isomers (i.e. cis-trans isomers), optical isomers or stereoisomers, such as diastereomers, as well as tautomers. Accordingly, it should be understood that their definition includes each and every individual isomer, including cis-trans isomers, stereoisomers and tautomers, as well as racemic mixtures of these and pharmaceutically acceptable salts thereof. Hence, the definition of the cationic polymers and the anionic copolymers comprising substructure I, II, and III is also intended to encompass all R- and S-isomers of a chemical structure in any ratio, e.g. with enrichment (i.e. enantiomeric excess or diastereomeric excess) of one of the possible isomers and corresponding smaller ratios of other isomers. In the particular case of amino acids, they may acquire L-configuration or D-configuration.

.. The anionic copolymers comprising substructure I, II, and III may be provided in any form suitable for the intended administration, in particular including pharmaceutically acceptable salts thereof.

.. Pharmaceutically acceptable salts refer to salts of both the cationic polymers or the anionic copolymers comprising substructure I, II, and III, which are considered to be acceptable for clinical, veterinary and/or cosmetic use. Typical pharmaceutically acceptable salts include those salts prepared by reaction of the cationic polymers or the anionic copolymers comprising substructure I, II, and III, and a mineral or organic acid or an organic or inorganic base. Such salts are known as acid addition salts and base addition salts, respectively. It will be recognized that the particular counter-ion or multiple counter-ions forming a part of any salt is not of a critical nature, so long as the salt as a whole is pharmaceutically acceptable and as long as the counter-ion does not contribute undesired qualities to the salt as a whole. These salts may be prepared by methods known to the skilled person.

.. Examples of pharmaceutically acceptable addition salts include acid addition salts formed with inorganic acids e.g. hydrochloric, hydrobromic, sulfuric, nitric, hydroiodic, metaphosphoric, or phosphoric acid; and organic acids e.g. succinic, maleic, acetic, fumaric, citric, tartaric, benzoic, trifluoroacetic, malic, lactic, formic, propionic, glycolic, gluconic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), ethanesulfonic, pantothenic, stearic, sulfinilic, alginic and galacturonic acid; and arylsulfonic, for example benzenesulfonic, p-toluenesulfonic, oxalic, methanesulfonic or naphthalenesulfonic acid; and base addition salts formed with alkali metals and alkaline earth metals and organic bases such as N,N-dibenzylethylenediamine, chloroprocaine, choline, diethanolamine, ethylenediamine, meglumine (N-methylglucamine), lysine and procaine; and internally formed salts.

.. In accordance with a particular embodiment, optionally in combination with any of the embodiments provided above or below, the polymer complex is obtained when mixed in aqueous medium at a pH ranging from 4-9, preferably in a pH ranging from 4.5-8.5, more preferably from 5-7.5, being particularly preferred from 6.5-7.4. The pH may be easily adjusted using a buffering solution as the solvent.

.. In accordance with a particular embodiment, optionally in combination with any of the embodiments provided above or below, the ionic strength of the solution to be mixed may be appropriately adjusted in a range that does not destroy the structure of the nanoparticles or inhibit encapsulation of the substance to be encapsulated in the nanoparticles, and it is preferably within the range from 0-1000 mM, preferably from 0-300 mM, more preferably from 0-150 mM, and being particularly preferred from 0-50 mM.

.. The polymer complex may have a particle hydrodynamic diameter ranging from 10 nm to 2000 nm, preferably from 20 nm to 800 nm, more preferably from 25 nm to 350 nm, from 30 nm to 300 nm, and from 30 nm to 200 nm, as measured by Dynamic Light Scattering.

.. The protein-based complex may have a particle hydrodynamic diameter ranging from 2 nm to 2000 nm, preferably from 5 nm to 1000 nm, more preferably from 10 nm to 800 nm, from 15 nm to 700 nm, and from 20 nm to 600 nm, as measured by Dynamic Light Scattering.

.. In the context of the present disclosure, the term "polyplex" refers to the polymer complex formed by electrostatic interaction between a cationic polymer and at least one polyanionic genetic material (preferably a nucleic acid) as described herein. In the context of the present disclosure the term "stabilized polyplex" or "shielded polyplex" refers to the polymer complex formed by electrostatic interaction between a cationic polymer, anionic shielding polymer and at least one polyanionic genetic material (preferably a nucleic acid) as described herein.

.. In accordance with a more particular embodiment, optionally in combination with any of the embodiments provided above or below, the N/P ratio in the polyplexes of the disclosure, which is defined as [total number (N) of cationic groups in the block copolymer] / [total number (P) of phosphate groups in the nucleic acid] is ranging from 1 to 100, preferably from 2 to 50, and more preferably from 2 to 30. The N/P ratio means a ratio between the molar concentration (N) of protonable amino groups derived from the side chain of the cationic polymer and the molar concentration (P) of phosphate groups derived from the nucleic acid in the mixed solution.

.. Examples of cationic polymers include poly-L-lysine (PLL), Poly-L-Ornithine (PLO), Poly-L-Histidine, polyamidoamine, polyarginine, poly-[2-{(2-aminoethyl)amino-ethyl-aspartamide] (pAsp(DET)), poly(dimethylaminoethyl methacrylate) (pDMAEMA), polyethyleneimine (PEI), chitosan, poly(beta-amino esters), cationic or cationically ionizable lipids or lipid-like materials, polycationic combinations of block, random or graft based on polyaminoacids such as a block co-polymer of polyethylene glycol and polyarginine, a block co-polymer of polyethylene glycol and polylysine, and a block co-polymer of polyethylene glycol and poly-[2-{(2-aminoethyl)amino}-ethyl-aspartamide] (PEG-pAsp(DET)), and any other appropriate cationic polymer as long as the cationic polymer can form complexes with at least one anionic active ingredient, and then this complex can form a polymer complex with the at least one shielding anionic copolymer comprising a substructure of formula I, II, or III as defined in the present invention.

.. Using appropriate surface functionality, the compounds of the present disclosure may be further decorated with a cell-targeting group and/or permeation enhancers that can actively target cells and aid in cellular entry, resulting in a conjugate which has improved cell-specific delivery. Optionally, the compounds of the present disclosure may be further decorated with a labelling or imaging agent that facilitates the visualization and/or detection.

.. Thus, in one embodiment, optionally in combination with any of the embodiments provided above or below, at least one cell-targeting agent; at least one labelling or imaging agent; or at least one cell-targeting agent and at least one labelling or imaging agent is covalently linked to the polypeptidic backbone of the cationic polymer or to the anionic copolymer, through an amino acid side residue, C or N-terminal groups via amide, ester, anhydride bonding or through a linker that include one or more functional groups, including without limitation, alkynes, azides, reactive disulfides, maleimides, hydrazide, hydrazones, Schiff bases, acetal, aldehydes, carbamates, and reactive esters.

.. The term "cell-targeting agent " refers to any biological or chemical structure displaying affinity for a molecule present in the human or animal body, which are able to direct the functionalized nanoparticles by directing them towards the target site for therapeutic treatment since e.g., it selectively binds to receptors that are expressed or over-expressed on specific cell types. The term therefore includes ligands for specific receptors or antigens, such as antibodies for a specific antigen, folic acid for its receptor or sugars such as galactose for its hepatic receptors. The targeting agent may be attached to the functionalized end-group of the anionic polymer through the A and/or A' moiety; or it may also be attached to the cationic polymer.

.. Cell-targeting groups are well known in the art. Examples of targeting agents include, but are not limited to monoclonal and polyclonal antibodies (e.g. IgG, IgA, IgM, IgD, IgE antibodies), fragment antibodies, nanobodies, sugars (e.g. mannose, mannose-6-phosphate, galactose, galactosamine, mannosamine), proteins (e.g. transferrin), oligopeptides (e.g. cyclic and acylic RGD-containing oligopeptides), oligonucleotides (e.g. aptamers), and vitamins (e.g. folate), a Her-2 binding peptide, TLR agonists, β-D-Glucose, Asn-Gly-Arg peptide, angiopep2, aptamers (A-9, A10, Anti-gp120, TTA1, sgc8, Anti MUC-1, AS1411), primaquine, zidovudine, superoxide dismutase, prednisolone, platinum, cisplatin, sulphamethoxazole, amoxicillin, etoposide, mesalzine, doxorubicin, paclitaxel, 5-amino salicylic acid, denosumab, docetaxel, calcitonin, proanthocyanidin, methotrexate, camptothecin, galactose, glycyrrhetinic acid, lactose, hyaluornic acid, octeotride, lactobionic acid, β-galactosyl moiety, arabino-galactan, chitosan, azo-based poly-phosphazene, azo group and 4-amino-benzyl-carbamate, succinate, 4,4'-dihydroxyazo benzene-3-carboxilic acid, cyclic RGD penta-peptide, Aspartic acid octapeptide, alendronate, transferrin, bisphosphonate adendronate, mono sialoganglioside GM1, gluthatione, E-selectinthioaptamer, poloxamer-407, a urokinase-type plasminogen activator receptor (uPAR) antagonist, a CXCR4 chemokine receptor antagonist, a GRP78 peptide antagonist, an RGD peptide, an RGD cyclic peptide, a luteinizing hormone-releasing hormone (LHRH) antagonist peptide, an aminopeptidase targeting peptide, a brain homing peptide, a kidney homing peptide, a heart homing peptide, a gut homing peptide, an integrin homing peptide, an angiogencid tumor endothelium homing peptide, an ovary homing peptide, a uterus homing peptide, a sperm homing peptide, a microglia homing peptide, a synovium homing peptide, a urothelium homing peptide, a prostate homing peptide, a lung homing peptide e.g. RCPLSHSLICY), laminin receptor binding peptide (e.g. YIGSR) a skin homing peptide, a retina homing peptide, a pancreas homing peptide, a liver homing peptide, a lymph node homing peptide, an adrenal gland homing peptide, a thyroid homing peptide, a bladder homing peptide, a breast homing peptide, a neuroblastoma homing peptide, a lymphoma homing peptide, a muscle homing peptide, a wound vasculature homing peptide, an adipose tissue homing peptide, a virus binding peptide, or a fusogenic peptide.

.. As used herein, the term "label or imaging" refers to a molecule that facilitates the visualization and/or detection of a targeting molecule disclosed herein. Thus, in the context of the present disclosure the expression, "labelling or imaging agent" refers to any substance that is used as a label, or that enhances specific structures in any imaging technique. An imaging agent, hence, includes optical imaging agent, magnetic resonance imaging agent, radioisotope, and contrast agent. Imaging or labelling agents are well known in the art. Particular examples or imaging or labelling agents are gases such as sterilized air, oxygen, argon, nitrogen, fluorine, perfluorocarbons, carbon dioxide, nitrogen dioxide, xenon and helium; commercially available agents used in positron emission tomography (PET), computer assisted tomography (CAT), single photon emission computerized tomography, x-ray, fluoroscopy, and magnetic resonance imaging (MRI). Examples of suitable materials for use as contrast agents in MRI include the gadolinium chelates currently available, such as diethylene triamine pentacetic acid (DTP A) and gadopentotate dimeglumine, as well as iron, magnesium, manganese, copper and chromium. Examples of materials useful for CAT and x-rays include iodine based materials for intravenous administration, such as ionic monomers typified by diatrizoate and iothalamate, non-ionic monomers such as iopamidol, isohexol, and ioversol, non-ionic dimers, such as iotrol and iodixanol, and ionic dimers, for example, ioxagalte. Other useful materials include barium for oral use and non-soluble salts such as zinc acetate. In some molecules, an imaging agent is a dye. In some molecules, an imaging agent is a fluorescent moiety. In some molecules, a fluorescent moiety is selected from: a fluorescent protein, a fluorescent peptide, a fluorescent dye, a fluorescent material or a combination thereof. Examples of fluorescent dyes include, but are not limited to, xanthenes (e.g., rhodamines, rhodols and fluoresceins, and their derivatives); bimanes; coumarins and their derivatives (e.g., umbelliferone and aminomethyl coumarins); aromatic amines (e.g., dansyl; squarate dyes); benzofurans; fluorescent cyanines; indocarbocyanines; carbazoles; dicyanomethylene pyranes; polymethine; oxabenzanthrane; xanthene; pyrylium; carbostyl; perylene; acridone; quinacridone; rubrene; anthracene; coronene; phenanthrecene; pyrene; butadiene; stilbene; porphyrin; pthalocyanine; lanthanide metal chelate complexes; rare-earth metal chelate complexes; and derivatives of such dyes. Examples of fluorescein dyes include, but are not limited to, 5-carboxyfluorescein, fluorescein-5-isothiocyanate, fluorescein-6-isothiocyanate and 6-carboxyfluorescein. Examples of rhodamine dyes include, but are not limited to, tetramethylrhodamine-6-isothiocyanate, 5-carboxytetramethylrhodamine, 5-carboxy rhodol derivatives, tetramethyl and tetraethyl rhodamine, diphenyldimethyl and diphenyldiethyl rhodamine, dinaphthyl rhodamine, rhodamine 101 sulfonyl chloride (sold under the tradename of TEXAS RED^{®}). Examples of cyanine dyes include, but are not limited to, Cy3, Cy3B, Cy3.5, Cy5, Cy5.5, Cy7, IRDYE680, Alexa Fluor 750, IRDye800CW, ICG. Examples of fluorescent peptides include GFP (Green Fluorescent Protein) or derivatives of GFP (e.g., EBFP, EBFP2, Azurite, mKalama1, ECFP, Cerulean, CyPet, YFP, Citrine, Venus, YPet). Fluorescent labels are detected by any suitable method. For example, a fluorescent label may be detected by exciting the fluorochrome with the appropriate wavelength of light and detecting the resulting fluorescence, e.g., by microscopy, visual inspection, via photographic film, by the use of electronic detectors such as charge coupled devices (CCDs), photomultipliers, etc. In some molecules, the imaging agent is labelled with a positron-emitting isotope (e.g.,18F) for positron emission tomography (PET), gamma-ray isotope (e.g., 99mTc) for single photon emission computed tomography (SPECT), or a paramagnetic molecule or nanoparticle (e.g.,Gd3+ chelate or coated magnetite nanoparticle) for magnetic resonance imaging (MRI). In some molecules, the imaging agent is labelled with: a gadolinium chelate, an iron oxide particle, a super paramagnetic iron oxide particle, an ultra-small paramagnetic particle, a manganese chelate or gallium containing agent. Examples of gadolinium chelates include, but are not limited to diethylene triamine pentaacetic acid (DTPA), 1,4,7,10-tetraazacyclododecane-1,4,7,10-tetraacetic acid (DOTA), and 1,4,7-triazacyclononane-N,N',N"-triacetic acid (NOTA). In some molecules, the imaging agent is a near-infrared fluorophore for near-infra red (near-IR) imaging, a luciferase (firefly, bacterial, or coelenterate) or other luminescent molecule for bioluminescence imaging, or a perfluorocarbon-filled vesicle for ultrasound. In some molecules, the imaging agent is a nuclear probe. In some molecules, the imaging agent is a SPECT or PET radionuclide probe. In some molecules, the radionuclide probe is selected from: a technetium chelate, a copper chelate, a radioactive fluorine, a radioactive iodine, a indium chelate. Examples of Tc chelates include, but are not limited to HYNIC, DTPA, and DOTA. In some molecules, the imaging agent contains a radioactive moiety, for example a radioactive isotope such as 211At, 131I, 125I, 90Y, 186Re, 188Re, 153Sm, 212Bi, 32P, 64Cu radioactive isotopes of Lu, and others.

.. In one embodiment, optionally in combination with any of the embodiments provided above or below, at least one active agent is covalently linked to the polypeptidic backbone of the cationic polymer or the anionic polymer through an amino acid side residue, C or N-terminal groups via amide, ester, anhydride bonding or through a linker that include one or more functional groups, including without limitation, alkynes, azides, reactive disulfides, maleimides, hydrazide, hydrazones, Schiff bases, acetal, aldehydes, carbamates, and reactive esters. In an alternative embodiment the covalent link is a bioresponsive one.

.. In another preferred embodiment, optionally in combination with any of the embodiments provided above or below, at least one active agent is linked to the polypeptidic backbone of the cationic polymer or the anionic polymer through electrostatic interaction.

.. In accordance with a particular embodiment, optionally in combination with any of the embodiments provided above or below, at least one active agent is selected from the group consisting of low molecular weight drugs, peptides, antibodies, hormones, enzymes, nucleic acids, proteins, and combinations thereof.

.. In accordance with a particular embodiment, optionally in combination with any of the embodiments provided above or below, the active ingredient is a nucleic acid, thus the positively charged nanoparticle is named herein polyplex. In a particular embodiment, the positively charged nanoparticle comprise a combination of two or more nucleic acids.

.. As used herein, the term "nucleic acid" refers to DNA or RNA. In a particular embodiment, optionally in combination with any of the embodiments provided above or below, the nucleic acid is an DNA/RNA hybrid, a short interfering RNA (siRNA), a microRNA (miRNA), a single guide RNA (sgRNA), a donorDNA, a self-amplyfing/replicating RNA, a circularRNA (oRNA), a plasmid DNA (pDNA), a linear-closed DNA (IcDNA), a short hairpin RNA (shRNA), messenger RNA (mRNA), and antisense RNA (aRNA), a messenger RNA (mRNA), a CRISPR guide RNA, an antisense nucleic acid, a decoy nucleic acid, an aptamer, and a ribozyme to name a few, and encompasses both the nucleotide sequence and any structural embodiments thereof, such as double stranded, single stranded, helical, hairpin, etc, and may contain modified or unmodified bases.

.. When distinct nucleic acids are provided, they may be all DNA molecules or all RNA molecules or may be mixtures of DNA and RNA molecules or molecules comprising an association of DNA and RNA strands.

.. The nucleic acid may be a poly- or oligonucleotide, such as oligo- or poly-double stranded RNA, oligo- or poly-double stranded DNA, oligo- or poly-single stranded RNA, oligo- or poly-single stranded DNA. Each of the nucleotides contained in the nucleic acid may be a naturally occurring nucleotide or a chemically-modified, non-naturally occurring nucleotide.

.. The strand length of the nucleic acid is not particularly limited, and the nucleic acid may have a short strand ranging from 10 to 200 bases, preferably from 20 to 180 bases, preferably from 25 to 100 bases, and preferably from 30 to 50 bases; or the nucleic acid may have a relatively long strand of from 200 to 20000 bases, more preferably of from 250 to about 15000 bases.

.. In accordance with a particular embodiment, optionally in combination with any of the embodiments provided above or below, the nucleic acid is linear-closed DNA (IcDNA), i.e. molecules wherein the double stranded region is flanked and protected by two single stranded loops thereby generating dumbbell-shaped molecules.

.. In a more particular embodiment, optionally in combination with any of the embodiments provided above or below, the IcDNA consists of a stem region comprising a double stranded DNA sequence of interest covalently closed at both ends by hairpin loops, the IcDNA comprising at least two modified nucleotides.

.. As used herein, the term "closed linear DNA" or "IcDNA" refers to a single stranded covalently closed DNA molecule that forms a "dumbbell" or "doggy-bone" shaped structure under conditions allowing nucleotide hybridization. Therefore, although the IcDNA is formed by a single stranded DNA molecule, the formation of the "dumbbell" structure by the hybridization of two complementary sequences within the same molecule generates a structure consisting of a double-stranded middle segment flanked by two single-stranded loops. Those skilled in the art knows how to generate IcDNA from open or closed double stranded DNA using routine molecular biology techniques. For instance, those skilled in the art knows that a IcDNA may be generated by attaching hairpin DNA adaptors -for instance, by the action of a ligase- to both ends of an open double stranded DNA. "Hairpin DNA adaptor" refers to a single stranded DNA that forms a stem-loop structure by the hybridization of two complementary sequences, wherein the stem region formed is closed at one end by a single stranded loop and is open at the other end.

.. A "modified nucleotide" is any nucleotide (e.g., adenosine, guanosine, cytidine, uracil and thymidine) that has been chemically modified -by modification of the base, the sugar or the phosphate group- or that incorporates a non-natural moiety in its structure. Thus, the modified nucleotide may be naturally or non-naturally occurring depending on the modification.

.. In accordance with an embodiment, optionally in combination with any of the embodiments provided above or below, the -/+ ratio between the positively charged nanoparticle or the positively charged protein and the at least one shielding anionic copolymer comprising substructure I, II, or III according to the present invention, which is defined as [total number negative charges (-) derived from polyanionic block in the shielding polymer(s)] / [total negative charges (-) derived from cationic groups in the block copolymer] is ranging from 0 to 2, preferably from 0.1 to 2, more preferably from 0.3 to 2, more preferably from 0.5 to 1.75, more preferably from 0.75 to 1.5. The -/+ ratio means a ratio between the molar concentration of negative charges (-) derived from the anionic block in the mixed solution and the molar concentration of positive charges (+) derived of protonable amino groups derived from the side chain of the cationic polymer or positively charged protein.

.. In one embodiment, optionally in combination with any of the embodiments provided above or below, the polymer complexes may comprise an amount of the at least an active agent in the range of 1 to 70% w/w based on the mass ratio of the active agent to the polymer complex. In a preferred embodiment, the range is of 1 to 60% w/w. In a still more preferred embodiment, the polymer complex comprises an amount of the active agent in the range of 2 to 50% w/w. Other preferred ranges are 2-40% w/w, 3-35% w/w, and 3-30% %w/w.

.. In a more particular embodiment, optionally in combination with any of the embodiments provided above or below, the protein-based complexes may comprise an amount of the protein in the range of 5 to 99% w/w based on the mass ratio of the active agent to the protein-based complexes. In a preferred embodiment, the range is of 15 to 98% w/w. In a still more preferred embodiment, the protein-based complex comprises an amount of the protein in the range of 30 to 95% w/w. Other preferred ranges are 40-92% w/w, 50-90% w/w, and 60-90 %w/w.

.. The polymer complexes or the protein-based complexes of the present disclosure constitute a useful tool for therapeutic or diagnostic indications, wherein the at least one anionic copolymer comprising the substructure I, II, or III act as a protective shielding for the positively charged nanoparticle bearing the active ingredient or the positively charged protein resulting in an improvement of certain properties such as increase of circulation times, safety or toxicological profile or the release profile in physiological conditions, and, in case of polyplex, also transfection efficiency to the desired cells,

.. The pharmaceutical, diagnostic or theragnostic compositions according to the disclosure, may be prepared in solid form or aqueous suspension, in a pharmaceutically acceptable diluent. These preparations may be administered by any appropriate administration route, for which reason said preparation will be formulated in the adequate pharmaceutical form for the selected administration route. In a more particular embodiment, optionally in combination with any of the embodiments provided above or below, administration is performed by oral, topical, rectal or parenteral route (including subcutaneous, intraperitoneal, intradermal, intramuscular, intravenous route, etc.). The pharmaceutical, diagnostic or theragnostic compositions may be applied to various types of animals, including human. Various conditions such as a dose, the number of doses and an administration period may be determined, as appropriate, depending on the type of a animal and the condition thereof.

.. The aforementioned pharmaceutical, diagnostic or theragnostic compositions may be prepared according to a common method by selecting and using, as appropriate, agents that are commonly used in drug manufacturing, such as an excipient, a filler, an extender, a binder, a wetting agent, a disintegrator, a lubricant, a surfactant, a dispersant, a buffer, a preservative, a solubilizer, an antiseptic, correctives, a soothing agent, a stabilizer, and an isotonizing agent. As the form of such a pharmaceutical composition, intravenous injection (including drops) is generally adopted. For example, the pharmaceutical composition of the present invention is provided in the form of a single dose ampule or a multidose container.

.. When the method refers to the diagnostics, this aspect could also be formulated as a method for the diagnosis of a disease in an isolated sample of a subject, the method comprises administering to said subject an effective amount of the any of the polymer complex, or pharmaceutical composition having one or more imaging agents as defined above to the isolated sample of the subject. The detection of these imaging agents may be carried out by well-known techniques such as imaging diagnostic techniques. Examples of imaging diagnostic techniques suitable for the present disclosure include, but not limited to, are ultrasound imaging, magnetic resonance imaging (MRI), fluoroscopy, X-ray, positron emission tomography (PET), single-photon emission computed tomography (SPECT), fluorescence microscopy, and in vivo fluorescence.

.. Thus, the disclosure also refers to the use of the polymer complex or the pharmaceutical composition of the disclosure as a bioimaging tool; particularly to track internalization and delivery of active agents or imaging agents.

.. As "bioimaging tool" is to be understood according to this description a reagent used in an imaging technique used in biology to trace some compartments of cells or particular tissues. Examples of bioimaging tools include chemiluminescent compounds, fluorescent and phosphorescent compounds, X-ray or alpha, beta, or gamma-ray emitting compounds, etc.

.. An additional aspect of the present disclosure relates to the use of the polymer complexes as defined herein, as non-viral vectors of general use for biomedical applications, such as vaccines or gene therapy, being effective for transfection of hosts eukaryotic cells in culture, in vivo or ex vivo, monocellular parasites and bacteria, including gene editing using the CRISP/Cas9 methodology.

.. An additional aspect of the present disclosure relates to the use of the protein-based complexes as defined herein, as carriers of general use in protein-based therapy, such as vaccines and protein replacement therapies.

.. In a particular embodiment, optionally in combination with any of the embodiments provided above or below, the present invention refers to the use of the polymer complexes as defined herein, as transfection reagents for delivering active agents (preferably nucleic acids regardless of size and structure, circular and linear nucleic acids) to target cells, in in vivo, in vitro or ex vivo. In a particular embodiment, optionally in combination with any of the embodiments provided above or below, the active agent is selected from the group consisting of low molecular weight drugs, peptides, proteins, antibodies, nucleic acids, aptamers, and combinations thereof.

.. Said transfection reagents are also useful for co-transfection of two or more active agents simultaneously, e.g. two or more nucleic acids, simultaneously. Transfection compositions (such as kits), as well as methods of using the transfection reagents to deliver nucleic acid to target cells are also within the scope of the present invention. Further embodiments will be apparent upon review of the disclosure.

.. The present invention also relates to a method for in vitro, ex vivo and in vivo transferring active agents comprising using a polymer complex as disclosed herein.

.. The present invention also provides compositions for use as pharmaceutical compositions for inducing a regulating effect on the expression of one or more target proteins responsible or involved in genetic hereditary diseases or complex genetic diseases, immune diseases, cancers, viral infections in various tissues/organs or tumors.

.. The present invention also relates to the in vitro or ex vivo use of compositions according to the invention in the production of biologics, in particular biologics encoding a recombinant protein, a peptide or an antibody; or in the production of recombinant virus, such as adeno-associated virus (AAV), lentivirus (LV), adenovirus, oncolytic virus, or baculovirus, or viral or virus-like particles, said compositions comprising a polymer complex as defined herein, comprising at least one nucleic acid molecule for transfection. As used herein, the term "biologics" refers to proteins or nucleic acids or combinations thereof, living entities such as cells or viruses, cell compartments, organoids, and tissues.

.. The present invention also relates to an in vitro or ex vivo use of the polymer complexes according to the invention for genome engineering, for cell reprogramming, for differentiating cells or for gene-editing.

.. The compositions for transfecting cells comprise a polymer complex as defined herein and an acceptable excipient, buffering agent, cell culture medium, or transfection medium.

.. The present invention is also directed to the compositions as defined herein for use as a therapeutic or prophylactic vaccine against viral infections, or a therapeutic vaccine against cancers. Generally, in this aspect, the vaccine is delivered through direct administration such as systemic, intramuscular, intradermal, intraperitoneal, intratumoral, oral, topical, or sub-cutaneous administration, and, in said vaccine, the composition is in association with a pharmaceutically acceptable vehicle. In other words, the vaccine may be injected directly into the body, in particular in a human individual, for inducing a cellular and/or a humoral response.

.. The cell targeting is achieved through different mechanisms and depends on the nature and properties of the transfection reagent, method or protocol composition or formulation and the route of administration.

.. In a more particular embodiment, optionally in combination with any of the embodiments provided above or below, the present invention refers to the polymer complex for use in the prevention and/or treatment of different diseases such as neurodegenerative disorders, neurological diseases, cancer, infectious diseases, disorders related to aging, neuro-inflammation, demyelinating disorder, multiple sclerosis, ischemic disorders, immune disorder, inflammatory disorders, rare diseases, among others depending on the active agent it carries.

.. The compounds described in the present disclosure, their pharmaceutically acceptable salts and solvates, and the pharmaceutical compositions containing them may be used jointly with other, additional drugs, to provide combined therapy. Said additional drugs may be a part of the same pharmaceutical composition or, alternatively, may be provided in the form of a separate composition for simultaneous or non-simultaneous administration with the pharmaceutical composition comprising a compound with the formula (I), a pharmaceutically acceptable salt, stereoisomer or solvate thereof.

.. In one embodiment, the present invention provides a nucleic acid-delivering kit comprising the aforementioned polymer complex of the invention. This kit may be preferably used in gene therapy for various types of target cells such as cancer cells, etc. In the kit of the present invention, the preservation state of the block copolymer is not particularly limited. Taking into consideration their stability (preservative quality), usability, etc., the block copolymer may be preserved in the form of a solution, powders, etc.

.. The kit of the present invention may comprise other constituents, as well as the aforementioned polymer complex. Examples of such other constituents include various types of buffers, various types of nucleic acids (plasmid DNA, antisense oligo DNA, siRNA, etc.) to be introduced into cells, a buffer used for dissolution, various types of proteins, and instruction for use (manual for use).

.. All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

.. The term "disorder" as used herein is intended to be generally synonymous, and is used interchangeably with, the terms "disease," "syndrome," and "condition" (as in medical condition), in that all reflect an abnormal condition of the human or animal body or of one of its parts that impairs normal functioning, is typically manifested by distinguishing signs and symptoms.

.. The term "pharmaceutically acceptable carrier," "pharmaceutically acceptable excipient," "physiologically acceptable carrier," or "physiologically acceptable excipient" as used herein, refers to a pharmaceutically acceptable material, composition, or vehicle, such as a liquid or solid filler, diluent, excipient, solvent, or encapsulating material. Each component should be "pharmaceutically acceptable" in the sense of being compatible with the other ingredients of a pharmaceutical formulation. It should also be suitable for use in contact with the tissue or organ of humans and animals without excessive toxicity, irritation, allergic response, immunogenicity, or other problems or complications, commensurate with a reasonable benefit/risk ratio.

.. The term "cosmetically acceptable carrier" or "dermatological acceptable carrier" which is herein used interchangeably refers to that excipients or carriers suitable for use in contact with human skin without undue toxicity, incompatibility, instability, allergic response, among others.

.. The term "therapeutically acceptable" refers to those compounds which are suitable for use in contact with the tissues of patients without excessive toxicity, irritation, allergic response, immunogenicity, are commensurate with a reasonable benefit/risk ratio, and are effective for their intended use.

.. The terms "treat", "treating" and "treatment", as used herein, refers to ameliorating symptoms associated with a disease or disorder, including preventing or delaying the onset of the disease or disorder symptoms, and/or lessening the severity or frequency of symptoms of the disease or disorder.

.. As used herein, the term "protective group" is a grouping of atoms that when attached to a reactive group in a molecule masks, reduces or prevents that reactivity. Protective groups for carboxyl and amino groups are well known in the art. Suitable amine protecting groups known in the art may be used without limitation and examples thereof include acyl-based groups, carbamate-based groups, imide-based groups, sulfonamide-based groups, and the like. Among them, methyloxycarbonyl, benzyloxycarbonyl, p-methoxybenzyloxycarbonyl, t-butyloxycarbonyl (Boc), 9-fluorenylmethyloxycarbonyl (FMOC), allyloxycarbonyl (Alloc), 2,2,2-trichloroethoxycarbonyl group (Troc), benzoyl (Bz), benzyl (Bn), p-methoxybenzyl (PMB), 3,4-dimethoxybenzyl (DMPM), p-methoxyphenyl (PMP), tosyl (Ts), trimethylsilylethyloxycarbonyl (Teoc), benzhydryl, triphenylmethyl (Trityl), (4-methoxyphenyl)diphenylmethyl (MMT), dimethoxytrityl (DMT), and diphenylphosphino groups are preferable. Introduction of the protective group may be carried out under a basic condition by adding a protecting agent corresponding to each protective group, to the reaction solution after the reduction reaction.

.. In the present invention, the "subject" may be a mammal inclusive of human. The subject may be a healthy subject or a subject affected with some disease. In the invention, "treatment" refers to curing, preventing or inducing remission of a disease or a disorder or decreasing a progressing speed of a disease or a disorder. The treatment may be attained by administering a therapeutically effective amount of a pharmaceutical composition.

.. Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

.. Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

### General procedure for anionic copolymer preparation

.. Anionic shielding copolymers were prepared following general preparation method as described below.

The block-copolymers were obtained via ROP (Ring Opening Polymerization). In a first step Methionine N-carboxyanhydride (NCA) and Alanine-NCA were polymerized to obtain the copolymers, followed by the addition of the second block (Glutamic(OtBu) NCA. Then, the oxidation of methionine is performed by employing *tert*-butyl hydroperoxide (TBHP). The last step consists of the deprotection of the Glutamic(OtBu) in acidic conditions.

### Example 1: Synthesis of shielding block copolymers (PMet(O)-co-PAla)-b-PGluOtBu

### 1.1 General procedure for the polymerization of (PMet-co-PAla)-b-PGluOtBu

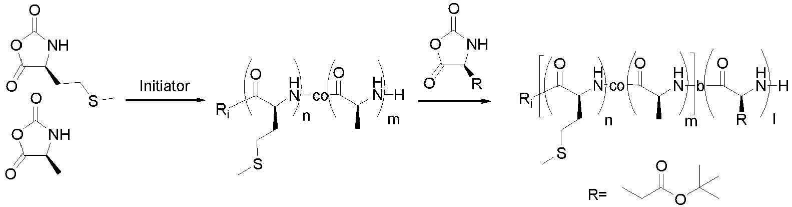

.. The block-copolymers were obtained via ROP (Ring Opening Polymerization). Methionine N-carboxyanhydride (NCA) and Alanine-NCA were added to a Schlenk tube fitted with a stirring bar and a stopper. After 3 cycles of vacuum/N₂, the mixture was dissolved in anhydrous THF. Then, the initiator (iPropylamine) diluted in THF (2 mL) and was added to the reaction mixture, which was stirred at RT for 3 days. Once NCA consumption was confirmed by IR the corresponding 2nd block aminoacid-NCA was added (Glutamic(OtBu) NCA to the reaction mixture dissolved in anhydrous THF. The mixture was stirred at RT for 2 days. Full conversion of the monomer was detected by IR. The reaction mixture was poured into diethyl ether to precipitate the product. The precipitate was lyophilized and the block copolymer was isolated as a white solid.

Yield: 80-98 %.

### 1.2. Oxidation of Methionine:

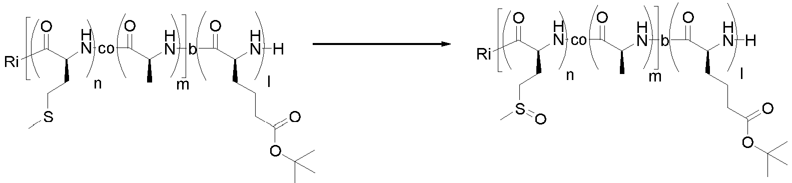

.. The oxidation reactions were carried out by suspending the block-copolymer in 16 equivalents of TBHP 80% (*tert*-butyl hydroperoxide) and 0.2 equivalents of CSA (camphorsulphonic acid) for each Met unit in MiliQ H₂O. The oxidation was quenched with Na₂S₂O₃ 0.1M and the product was purified by TFF (Tangential Flow Filtration), and lyophilized.

### 1.3 General procedure for Glu(OtBu) deprotection step:

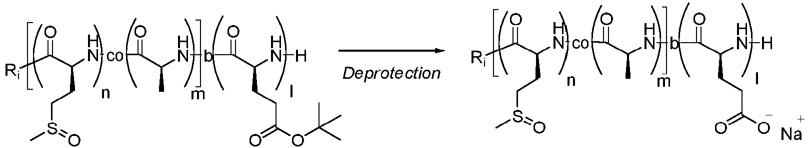

.. The block copolymer of PMet(O)-co-PAla-b-PGluOtBu, was dissolved in trifluoroacetic acid at 0°C (100 mg/mL) and the mixture was stirred at 5 °C for 1 hour. The reaction mixture was poured into diethyl ether to precipitate the product. The precipitate was isolated by centrifugation (3750 rpm, 4 min) and dried under vacuum. The block copolymer was isolated as a white solid.

Yield: 70-95 %.

[V1-V5]1H NMR (D2O): δ 1.50 (d, CH3 Ala), 1.90-2.49 (m, 2 CH2 Glu + CH2 Met(O)),2.85-3.20 (m, CH2 Met(O)), 4.25-4.65 (m, CH Met(O) + CH Ala + CH Glu).

**Table 1. Characterization of different block copolymers based on (PMet(O)co PAla)-b-PGlu(ONa) (Vn)**

| **Code** | **Initiator** | **DP (m) for Met(O)^{a}** | **DP (m) for Ala^{a}** | **DP (n) for Glu(ONa) ^{a}** | **Mw by SEC (KDa)** | **PDI** |
|---|---|---|---|---|---|---|
| **V1** | ⁱPropylamine | 138 | 19 | 65 | 26.825 ^{b} | 1.24 |
| **V2** | ⁱPropylamine | 167 | 25 | 82 | 28.821 ^{c} | - |
| **V3** | ⁱPropylamine | 154 | 25 | 84 | 37.782 ^{c} | - |
| **V4** | ⁱPropylamine | 139 | 20 | 98 | 39.235 ^{c} | - |
| **V5** | ⁱPropylamine | 167 | 25 | 80 | 34.770^{bc} | - |

| | | | | | | |
|---|---|---|---|---|---|---|
| **^{a}** Determined by NMR. ^{b}Determined by SEC-MALS. ^{c}Determined by SEC-column calibration. **V1** = iPr-P[Met(O)138-co-Ala19]-b-PGlu(ONa)65 **V2** = iPr-P[Met(O)167-co-Ala25]-b-PGlu(ONa)82 **V3** = iPr-P[Met(O)154-co-Ala25]-b-PGlu(ONa)84 **V4** = iPr-P[Met(O)139-co-Ala20]-b-PGlu(ONa)98 **V5** = iPr-P[Met(O)167-co-Ala25]-b-PGlu(ONa)80 wherein the cited DP numbers are subject to a reasonable uncertainty within the range ±20%. | | | | | | |

### Example 2. Synthesis and description of Polycationic carriers

.. In order to prove the stabilizing ability and the enhanced transfection features imbued to the polyplexes by the shielding polymers, the universal benchmark jetPEI^{®} was used (Polyplus-transfection S.A, Illkirch, France) (Ref Polyplus: 101-10N). JetPEI^{®} is a powerful reagent that ensures robust, effective and reproducible DNA transfection into mammalian cells with low toxicity. jetPEI^{®} is mainly composed of a linear polyethylenimine manufactured at Polyplus-transfection. jetPEI^{®} is provided as a 7.5 mM solution in sterile and apyrogenic water (expressed as concentration of nitrogen residues). Additionally, other commercial cationic polymers have been tested: nbu-Poly-L-Ornithine hydrobromide (PLO) (sourced from Polypeptide Therapeutic Solutions catalogue Batch number CM-CC1004-01-42B); chitosan (CHI) (purchased from Kytozyme (15-20 kDa)); linear Poly-L-Lysine (nbu-PLL) (sourced from Polypeptide Therapeutic Solutions Batch number CM-CC1030-02-08A) and Star-like Poly-L-Lysine (St-PLL) (sourced from Polypeptide Therapeutic Solutions Batch number PI03-01-166C). Also, to prove stabilization of very different architectural and compositional alternative polycations, different polycationic NVVs based on Star-shaped polyaminoacids were synthesized and its polyplexes were also stabilized and assayed. The following examples describe the preparation of the afore mentioned polycationic compounds used to complexate and transport the genetic material.

### Example 2.1 Preparation of compound N1

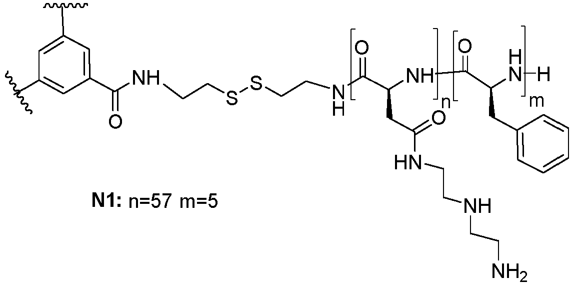

.. In general terms, to synthesize compounds of formula (N1) according to the present disclosure, first, the 3-arm star initiator was obtained within 2-3 steps. Such initiator was then used to polymerize y-benzyl L-Aspartate-NCA and L-Phenylalanine NCA, to yield the star random copolymer benzyl protected (St-PAsp(Bz)-co-PPhe). The benzyl groups were removed by an aminolysis reaction to yield the corresponding Star-PAsp-Oligoamine-co-PPhe.

Scheme 1 shows a particular example of polymerization and aminolysis steps:

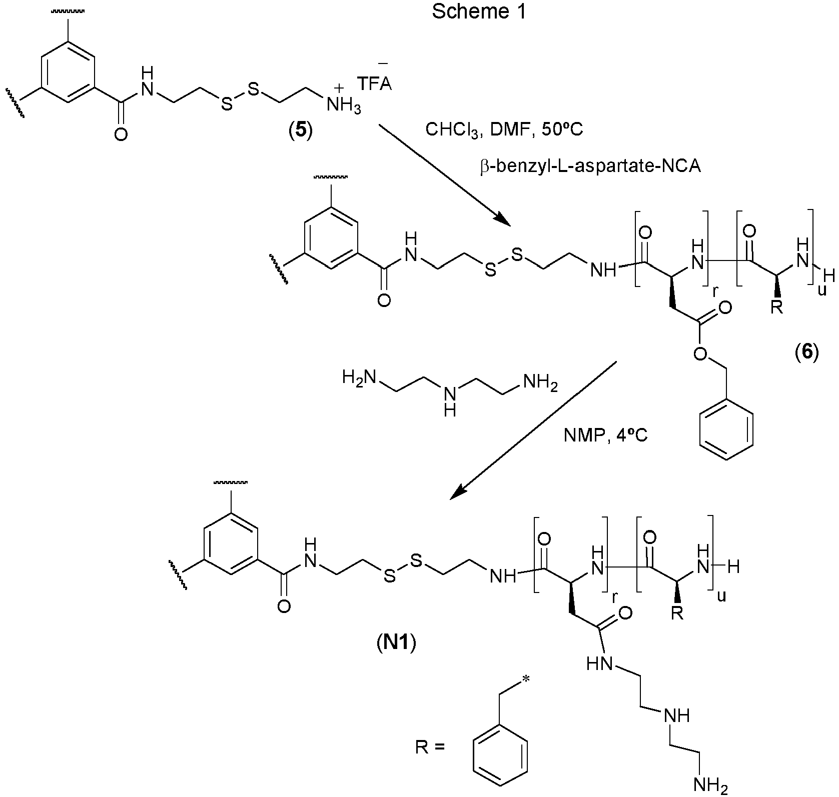

### 2.1.A Synthesis of 3-arm star initiators

.. The synthetic routes towards 3-arm star initiators is described below.

### 2.1.A.1: Trifluoroacetic acid salt of N,N,N-tris(2-((2-Aminoethyl) disulfanyl)ethyl)benzene-1,3,5-tricarboxamide (St-S-S-Initiator) (5)

.. Trifluoroacetic acid salt of N,N,N-tris(2-((2-Aminoethyl)disulfanyl)ethyl)benzene-1,3,5-tricarboxamide (St-S-S-initiator) (5) was synthesized following the general procedure disclosed Scheme 2.

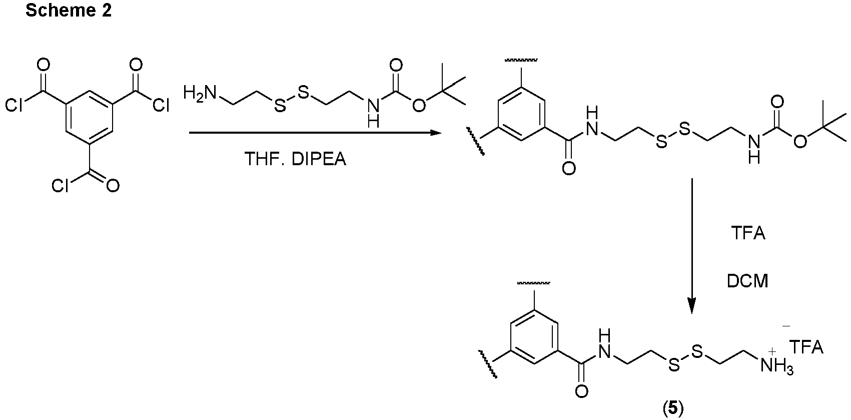

.. The synthesis of the trimeric amine initiator starts with a coupling reaction followed by amine deprotection.

### Step (a) Synthesis of tri-tert-butyl ((((benzenetricarbonyltris- (azanediyl))tris(ethane-2,1-diyl))tris(disulfanediyl))tris(ethane-2,1-diyl))- tricarbamate:

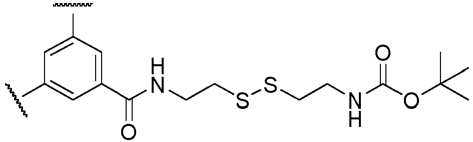

.. N-(tert-Butyloxycarbonyl)cystamine (7.99, 27 mmol, 3.3 eq) was weighed into a flame-dried two-neck round-bottom flask and was dissolved in 56 mL anhydrous THF. Freshly distilled DIPEA (4.75 mL, 27 mmol, 3.3 eq) was added and stirred for 15 min at room temperature. 1,3,5-Benzenetricarbonyl trichloride (2.25 g, 8.3 mmol, 1 eq) was weighed into a flame-dried two-neck round-bottom flask and was dissolved in 28 mL of anhydrous THF. The trichloride solution was slowly added to the N-(tert-butyloxycarbonyl)cystamine mixture via syringe. The progress of reaction was monitored by thin-layer chromatography (TLC). After 4h, the solvent was evaporated in vacuo and the residue was dissolved in ethyl acetate. The organic layer was sequentially washed with Milli-Q water, 1M hydrochloric acid and saturated sodium bicarbonate solution. The organic phase was dried over magnesium sulfate anhydrous and concentrated in vacuo afforded tri-tert-butyl ((((benzenetricarbonyltris-(azanediyl))tris(ethane-2,1-diyl))tris-(disulfanediyl))tris(ethane-2,1-diyl))-tricarbamate as a white foam (7.5 g, η= 98%).

¹H NMR (CDCl₃): δ = 1.39 (brs, 27H, -C(CH₃)₃), 2.84 (t, J = 6.26 Hz, 6H, CH₂), 2.96 (t, J = 6.84 Hz, 6H. CH₂), 3.46 (m, 6H, CH₂), 3.79 (m, 6H, CH₂), 5.18 (brs, 3H, - NHBoc), 7.39 (brs, 3H, aryl CH).

### Step (b): Synthesis of trifluoroacetic acid salt of N,N,N-tris(2-((2-aminoethyl)disulfanyl)ethyl)benzene-1,3,5-tricarboxamide(St-S-S-Initiator) (5):

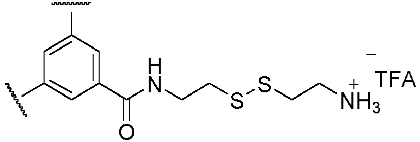

.. 7.5 g (8.19 mmol) of the initiator (5) was dissolved in anhydrous dichloromethane (180 mL) and 90 mL of TFA were added. The reaction was stirred under nitrogen atmosphere for 60 min and the completion of the reaction was monitored by TLC. The solvents were evaporated under vacuum. The TFA salt of the initiator (**5**) (7 g, 7.31 mmol) was obtained in quantitative yield and dried under vacuum.

1H NMR (D2O): δ = 2.86 (m, 12 H), 3.25 (t, J = 6.49 Hz, 8H), 3.60 (t, J = 6.85 Hz, 8H), 8.02 (brs, 3H, aryl CH).

### 2.1.B.Synthesis of Star-PAsp(Bz) (6) copolymers comprising a hydrophobic fragment.

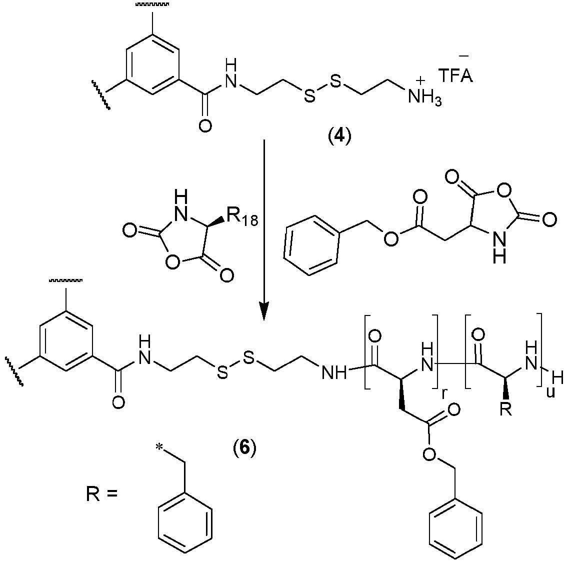

.. To synthesize the copolymers with hydrophobic residues, the polymerization was carried out via ring opening polymerization mechanism using trifluoroacetic acid salt of N,N,N-tris(2-((2-aminoethyl)disulfanyl)ethyl)-benzene-1,3,5-tricarboxamide as initiator.

### General procedure for synthesized St-S-S-PAsp(Bz)(45)-co-PPhe(5) (N1):

.. β-benzyl-L-aspartate-N-carboxy anhydride (3,5 g, 14,15 mmol) and L-Phenylalanine-N-carboxy anhydride (1,57 mmol) was added to a Schlenk tube fitted with a stirrer bar and a stopper, and purged with 3 cycles of vacuum/N₂, and dissolved in a mixture of anhydrous chloroform (100 mL) and DMF (6 mL). Then, the star initiator was dissolved in DMF (4 mL) and was added to the reaction mixture. The mixture was stirred at 50 °C for 16 hours. Upon completion, the reaction mixture became clear and full conversion of the monomer could be detected by IR. The reaction mixture was poured into diethyl ether to precipitate the product. The precipitate was isolated by centrifugation (3750 rpm, 4 min) and dried under vacuum. Copolymer was isolated as a white solid.

Yield: 70-80% 1H NMR (TFA): δ = 2.99 (s, 2H, CH), 3.94 (brs, 1H, CH), 4.93 (s, 1H, CH), 5.15 (m, 2H, benzyl CH2), 7.20 (s, 5H, aryl CH), 8.42 (s, aryl CH)..

.. The introduction of ratios of the repeating unit were adjusted changing the mixing ratios of the corresponding monomer units to be allowed to react. In this precursor, the hydrophobic residue matches by 1H-NMR with the protective group of the poly-aspartic. This system will be analyzed after the aminolysis reaction (next example 5.1.C).

### 2.1.C. Synthesis of amphiphilic polyaspartamide derivatives St-S-S-PAspDET-co-PR18 (10).

.. As shown in the synthetic route below, polyamino acids were prepared by the simultaneous aminolysis reaction of PBLA with DET.

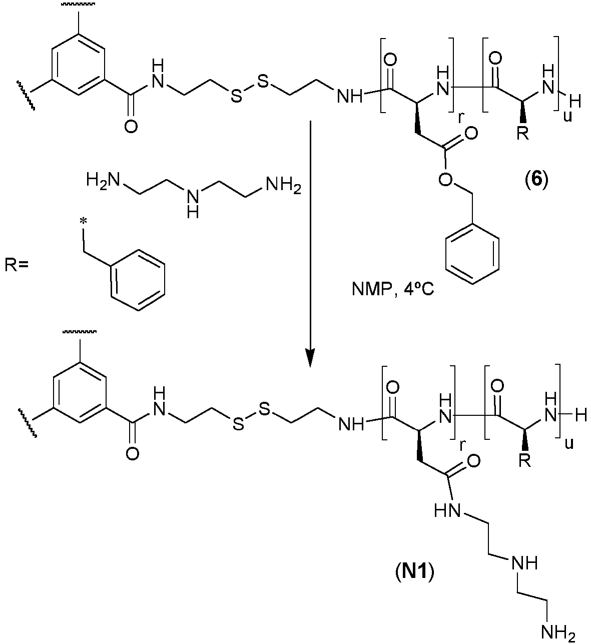

.. As an example, herein we describe the synthetic method in which R18 represents a phenylalanine group. Copolymer of St-S-SPAsp(Bz)45-co-PPhe(5) (500 mg of copolymer, 470 mg of PBLA, DP:45) was dissolved in NMP (10 mL) and cooled to 4 °C. The resultant copolymer solution was added dropwise to the mixture of DET (12 mL, 50 eq. vs unit of PAsp(Bz)) and the solution was stirred for 4 h at 4 °C under nitrogen atmosphere. After this time, the reaction mixture was added dropwise into cold HCl 6 M for neutralization (pH 3.5). The polymer product was purified by centrifugal-assisted ultrafiltration. After filtration, the remaining aqueous polymeric solution was lyophilized to obtain the final product.

Yield: 70-80%. ¹H NMR (D₂O) [R18= Phe side chain]: δ = 2.91 (brs, 2H, CH2), 3.84-3.18 (m, 2H, CH2), 7.34 (brs, 5H, aryl CH of Phe), 8.33 (s, aryl CH).

Table 2 refers to amphiphilic copolymer St-S-S-PAspDET-co-R18 according to formula (**N1**).

**Table 2. Characterization data of N1 polymer**

| **Compound** | **R** | **Ratio^{a} PAsp:R18** | **Mn^{b} (Da)** | **Ð** |
|---|---|---|---|---|
| **N1** | Phe | 57:5 | 13098 | 1.282 |

| | | | | |
|---|---|---|---|---|
| **^{a}** Determined by NMR. ^{b} Determined by SEC. Mn and DP refer to number average molar mass and degree of polymerization respectively. Ð represent polydispersity as determined by SEC-MALS software analysis. | | | | |

### Example 2.2 Preparation of compound N3

Analogously to the synthesis of St-S-S-PAspDET-co-R1 (N1), the same experimental procedure is used to generate St-S-S-PAspDET-co-PAsplmidazolamine (N3).

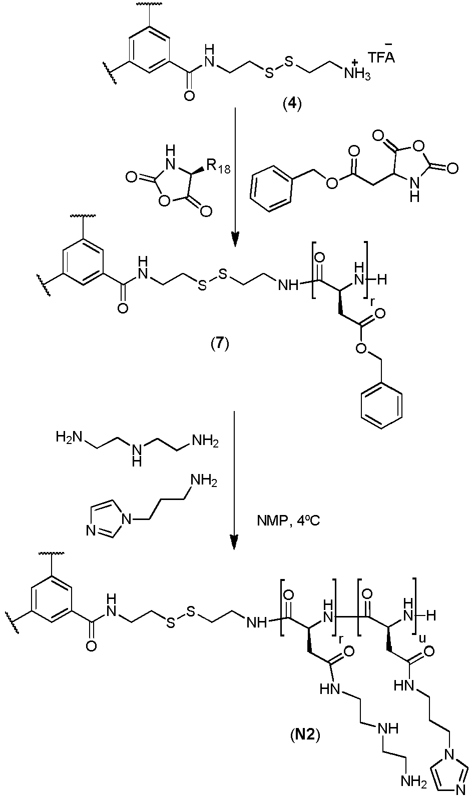

Synthesis of St-S-S-Poly(β-benzyl-L-aspartate) (Star-PAsp(Bz) (7) was carried out following the experimental procedure described in the previous section.

Yield: 70-90% ¹H NMR (TFA): δ = 2.92 (m, 2H, CH₂), 4.85 (s, 1H, CH), 5.05 (m, 2H, benzyl CH₂), 7.13 (s, 5H, aryl CH), 8.38 (s, aryl CH).

**Table 3. Shows the DP (degree of polymerization) obtained for different Star-PAsp(Bz) of formula (7).**

| **Compound** | **DP_{PAsp(Bz) theoretical}** | **Mn^{a} (kDa)** | **DP_{PAsp(Bz)}^{a}** |
|---|---|---|---|
| 7 | 100 | 23.9 | 112 |

| | | | |
|---|---|---|---|
| **^{a}** Determined by NMR. ^{b} Determined by SEC. Mn and DP refer to number average molar mass and degree of polymerization respectively. Ð represent polydispersity as determined by SEC-MALS software analysis. | | | |

The aminolysis reaction of poly(β-benzyl-L-aspartate) (**7**) to produce St-PAspDET-co-PAsplmidazolamine (N3) was carried out following the same experimental procedure as for N1 but in this case two types of amines are used: diethylenetriamine and 1-(3aminopropyl)imidazole.

1¹H NMR [St-PAspDET/lmidazolamine] (D₂O): δ = 2.14 (brs, 2H, CH₂), 2.87 (brs, 2H, CH2), 3.22 (m, 2H, CH₂), 4.30 (brs, 2H, CH₂),7.52 (s, Imidazole CH), 7.57 (s, Imidazole CH), 8.34 (s, aryl CH), 8.78 (s, Imidazole CH).

**Table 4. Characterization data of N3 polymer**

| **Compound** | **R₁** | **DP^{a}** | **MW^{b} (KDa)** | **Ð** |
|---|---|---|---|---|
| N3 | -CH-CH₂SSCH₂CH:₂ | R(86)/t(31) | 24.9 | 1.05 |

| | | | | |
|---|---|---|---|---|
| **^{a}** Determined by NMR. ^{b} Determined by SEC. Mn and DP refer to number average molar mass and degree of polymerization respectively. Ð represent polydispersity as determined by SEC-MALS software analysis. | | | | |

### Example 3 . Polyplex formulation

.. Polyplex formulations are named as "PX_{Nn_ratio1_Shielding polymer_ratio2}^{nuc"}, wherein "Nₙ" corresponds to the polycationic compound nomenclature as given herein (i.e. **N1** for the Star-PAsp-Oligoamine-co-PPhe cationic polymer as described in Example 2.1 above, **N2** for the benchmark jetPEI^{®}, and **N3** for the St-S-S-PAspDET-co-PAsplmidazolamine cationic polymer as described in Example 2.2 above), **N4** for the star-like poly-L-lysine (St-PLL Curapath catalogue, product #1075), **N5** for the linear poly-L-lysine (nbu-PLL, Curapath catalogue, product #1019), **N6** for which the nbu-poly-L-ornithine (PLO Curapath catalogue, product #1018), and **N7** for the chitosan (CHI, acquired from Kitozyme catalogue, chitosan 15-20 kDa) is used to form the polyplex; wherein "ratio1" refers to the N/P ratio of cationic polymer vs genetic material, "shielding polymer" refers to the polyanionic shielding diblock copolymer (**Vn**), "ratio2" refers to the +/- ratio of the cationic polymer vs shielding (anionic) polymer; and wherein "nuc" refers to the type of nucleic acid: pDNA, mRNA or IcDNA.

.. In the following examples, a pDNA (purchased from PlasmidFactory, with reference PF461 (pCMV-luc)), containing 6233 bp expressing luciferase) was employed; commercial mRNA(luc) (purchased form Trilink Biotechnologies), commercial pDNA(GFP) (obtained from PlasmidFactory GmbH & Co. KG) and a IcDNA (SEQ ID NO. 1) which was obtained according to standard molecular biology methods, such as the one disclosed in Heinrich, M. et al. "Linear closed mini DNA generated by the prokaryotic cleaving-joining enzyme TelN is functional in mammalian cells", J Mol Med, 2002, vol. 80, pp. 648-654.

.. The sequence of the IcDNA according to SEQ ID NO. 1, in the examples, is that of Table 5.

**Table 5. Sequence of the IcDNA (SEQ ID NO. 1)**

| |
|---|
| SEQ ID NO: 1; linear closed DNA (lcDNA) synthetic construct (artificial) with 5580 nucleotides. |
| gcgtataatg gactattgtg tgctgatagg tctcgccctg atttaaatcg tcgaccgtta 60 cataacttac ggtaaatggc ccgcctggct gaccgcccaa cgacccccgc ccattgacgt 120 caataatgac gtatgttccc atagtaacgc caatagggac tttccattga cgtcaatggg 180 tggagtattt acggtaaact gcccacttgg cagtacatca agtgtatcat atgccaagta 240 cgccccctat tgacgtcaat gacggtaaat ggcccgcctg gcattatgcc cagtacatga 300 ccttatggga ctttcctact tggcagtaca tctacgtatt agtcatcgct attaccatgg 360 tgatgcggtt ttggcagtac atcaatgggc gtggatagcg gtttgactca cggggatttc 420 caagtctcca ccccattgac gtcaatggga gtttgttttg gcaccaaaat caacgggact 480 ttccaaaatg tcgtaacaac tccgccccat tgacgcaaat gggcggtagg cgtgtacggt 540 gggaggtcta tataagcaga gctcgtttag tgaaccgtca gatcgcctgg agacgccatc 600 cacgctgttt tgacctccat agaagacacc gggaccgatc cagcctccgg actctagagg 660 atccggtact cgaggaactg aaaaaccaga aagttaactg gtaagtttag tctttttgtc 720 |
| ttttatttca ggtcccggat ccggtggtgg tgcaaatcaa agaactgctc ctcagtggat 780 gttgccttta cttctaggcc tgtacggaag tgttacttct gctctaaaag ctgcggaatt 840 gtacccgcgg cccgggatcc accggtcgcc accatggaag acgccaaaaa cataaagaaa 900 ggcccggcgc cattctatcc tctagaggat ggaaccgctg gagagcaact gcataaggct 960 atgaagagat acgccctggt tcctggaaca attgctttta cagatgcaca tatcgaggtg 1020 aacatcacgt acgcggaata cttcgaaatg tccgttcggt tggcagaagc tatgaaacga 1080 tatgggctga atacaaatca cagaatcgtc gtatgcagtg aaaactctct tcaattcttt 1140 atgccggtgt tgggcgcgtt atttatcgga gttgcagttg cgcccgcgaa cgacatttat 1200 aatgaacgtg aattgctcaa cagtatgaac atttcgcagc ctaccgtagt gtttgtttcc 1260 aaaaaggggt tgcaaaaaat tttgaacgtg caaaaaaaat taccaataat ccagaaaatt 1320 attatcatgg attctaaaac ggattaccag ggatttcagt cgatgtacac gttcgtcaca 1380 tctcatctac ctcccggttt taatgaatac gattttgtac cagagtcctt tgatcgtgac 1440 aaaacaattg cactgataat gaattcctct ggatctactg ggttacctaa gggtgtggcc 1500 cttccgcata gaactgcctg cgtcagattc tcgcatgcca gagatcctat ttttggcaat 1560 caaatcattc cggatactgc gattttaagt gttgttccat tccatcacgg ttttggaatg 1620 tttactacac tcggatattt gatatgtgga tttcgagtcg tcttaatgta tagatttgaa 1680 gaagagctgt ttttacgatc ccttcaggat tacaaaattc aaagtgcgtt gctagtacca 1740 accctatttt cattcttcgc caaaagcact ctgattgaca aatacgattt atctaattta 1800 cacgaaattg cttctggggg cgcacctctt tcgaaagaag tcggggaagc ggttgcaaaa 1860 cgcttccatc ttccagggat acgacaagga tatgggctca ctgagactac atcagctatt 1920 ctgattacac ccgaggggga tgataaaccg ggcgcggtcg gtaaagttgt tccatttttt 1980 gaagcgaagg ttgtggatct ggataccggg aaaacgctgg gcgttaatca gagaggcgaa 2040 ttatgtgtca gaggacctat gattatgtcc ggttatgtaa acaatccgga agcgaccaac 2100 gccttgattg acaaggatgg atggctacat tctggagaca tagcttactg ggacgaagac 2160 gaacacttct tcatagttga ccgcttgaag tctttaatta aatacaaagg atatcaggtg 2220 gcccccgctg aattggaatc gatattgtta caacacccca acatcttcga cgcgggcgtg 2280 |
| gcaggtcttc ccgacgatga cgccggtgaa cttcccgccg ccgttgttgt tttggagcac 2340 ggaaagacga tgacggaaaa agagatcgtg gattacgtcg ccagtcaagt aacaaccgcg 2400 aaaaagttgc gcggaggagt tgtgtttgtg gacgaagtac cgaaaggtct taccggaaaa 2460 ctcgacgcaa gaaaaatcag agagatcctc ataaaggcca agaagggcgg aaagtccaaa 2520 ttgtaaagcg gccgcgggga tccagacatg ataagataca ttgatgagtt tggacaaacc 2580 acaactagaa tgcagtgaaa aaaatgcttt atttgtgaaa tttgtgatgc tattgcttta 2640 tttgtaacca ttataagctg caataaacaa gttaacaaca acaattgcat tcattttatg 2700 tttcaggttc agggggaggt gtgggaggtt ttttagtcga ccatttaaat cggtctcgag 2760 ggtatcagca cacaattgcc cattatacgc gcgtataatg ggcaattgtg tgctgatacc 2820 ctcgagaccg atttaaatgg tcgactaaaa aacctcccac acctccccct gaacctgaaa 2880 cataaaatga atgcaattgt tgttgttaac ttgtttattg cagcttataa tggttacaaa 2940 taaagcaata gcatcacaaa tttcacaaat aaagcatttt tttcactgca ttctagttgt 3000 ggtttgtcca aactcatcaa tgtatcttat catgtctgga tccccgcggc cgctttacaa 3060 tttggacttt ccgcccttct tggcctttat gaggatctct ctgatttttc ttgcgtcgag 3120 ttttccggta agacctttcg gtacttcgtc cacaaacaca actcctccgc gcaacttttt 3180 cgcggttgtt acttgactgg cgacgtaatc cacgatctct ttttccgtca tcgtctttcc 3240 gtgctccaaa acaacaacgg cggcgggaag ttcaccggcg tcatcgtcgg gaagacctgc 3300 cacgcccgcg tcgaagatgt tggggtgttg taacaatatc gattccaatt cagcgggggc 3360 cacctgatat cctttgtatt taattaaaga cttcaagcgg tcaactatga agaagtgttc 3420 gtcttcgtcc cagtaagcta tgtctccaga atgtagccat ccatccttgt caatcaaggc 3480 gttggtcgct tccggattgt ttacataacc ggacataatc ataggtcctc tgacacataa 3540 ttcgcctctc tgattaacgc ccagcgtttt cccggtatcc agatccacaa ccttcgcttc 3600 aaaaaatgga acaactttac cgaccgcgcc cggtttatca tccccctcgg gtgtaatcag 3660 aatagctgat gtagtctcag tgagcccata tccttgtcgt atccctggaa gatggaagcg 3720 ttttgcaacc gcttccccga cttctttcga aagaggtgcg cccccagaag caatttcgtg 3780 taaattagat aaatcgtatt tgtcaatcag agtgcttttg gcgaagaatg aaaatagggt 3840 tggtactagc aacgcacttt gaattttgta atcctgaagg gatcgtaaaa acagctcttc 3900 |
| ttcaaatcta tacattaaga cgactcgaaa tccacatatc aaatatccga gtgtagtaaa 3960 cattccaaaa ccgtgatgga atggaacaac acttaaaatc gcagtatccg gaatgatttg 4020 attgccaaaa ataggatctc tggcatgcga gaatctgacg caggcagttc tatgcggaag 4080 ggccacaccc ttaggtaacc cagtagatcc agaggaattc attatcagtg caattgtttt 4140 gtcacgatca aaggactctg gtacaaaatc gtattcatta aaaccgggag gtagatgaga 4200 tgtgacgaac gtgtacatcg actgaaatcc ctggtaatcc gttttagaat ccatgataat 4260 aattttctgg attattggta attttttttg cacgttcaaa attttttgca accccttttt 4320 ggaaacaaac actacggtag gctgcgaaat gttcatactg ttgagcaatt cacgttcatt 4380 ataaatgtcg ttcgcgggcg caactgcaac tccgataaat aacgcgccca acaccggcat 4440 aaagaattga agagagtttt cactgcatac gacgattctg tgatttgtat tcagcccata 4500 tcgtttcata gcttctgcca accgaacgga catttcgaag tattccgcgt acgtgatgtt 4560 cacctcgata tgtgcatctg taaaagcaat tgttccagga accagggcgt atctcttcat 4620 agccttatgc agttgctctc cagcggttcc atcctctaga ggatagaatg gcgccgggcc 4680 tttctttatg tttttggcgt cttccatggt ggcgaccggt ggatcccggg ccgcgggtac 4740 aattccgcag cttttagagc agaagtaaca cttccgtaca ggcctagaag taaaggcaac 4800 atccactgag gagcagttct ttgatttgca ccaccaccgg atccgggacc tgaaataaaa 4860 gacaaaaaga ctaaacttac cagttaactt tctggttttt cagttcctcg agtaccggat 4920 cctctagagt ccggaggctg gatcggtccc ggtgtcttct atggaggtca aaacagcgtg 4980 gatggcgtct ccaggcgatc tgacggttca ctaaacgagc tctgcttata tagacctccc 5040 accgtacacg cctaccgccc atttgcgtca atggggcgga gttgttacga cattttggaa 5100 agtcccgttg attttggtgc caaaacaaac tcccattgac gtcaatgggg tggagacttg 5160 gaaatccccg tgagtcaaac cgctatccac gcccattgat gtactgccaa aaccgcatca 5220 ccatggtaat agcgatgact aatacgtaga tgtactgcca agtaggaaag tcccataagg 5280 tcatgtactg ggcataatgc caggcgggcc atttaccgtc attgacgtca atagggggcg 5340 tacttggcat atgatacact tgatgtactg ccaagtgggc agtttaccgt aaatactcca 5400 cccattgacg tcaatggaaa gtccctattg gcgttactat gggaacatac gtcattattg 5460 |
| acgtcaatgg gcgggggtcg ttgggcggtc agccaggcgg gccatttacc gtaagttatg 5520 taacggtcga cgatttaaat cagggcgaga cctatcagca cacaatagtc cattatacgc 5580 |

### 3.1. Polyplex formulation procedure 1.

.. Shielded polyplex formulations to study stability, size, toxicity and transfection capacity were prepared in-situ (mixing in a pipette) as follows:

.. The desired amount of pDNA, mRNA or IcDNA and the calculated amount of the cationic polymer at indicated charge-ratio (+/-) or amine to phosphate ratio (N/P) were diluted in separate tubes in PBS pH 7.4. Only protonatable nitrogens, not amide nitrogens, were considered in the +/- ratio and N/P ratio calculations. Prior to polyplex formation, the corresponding amount of shielding polymer was added and mixed to the nucleic acid test tube. For the shielded polyplex formation, the cationic polymer solution and the genetic material+shielding polymer solution were mixed by rapidly pipetting up and down (ten times) and incubated for 20 min at RT. Then the polyplexes formed were characterized by DLS to determine the size.

.. As **^{a}** particular example, the shielded polyplex PX_{N1_8_V1_1}^{pDNA} loaded with 20µg of pDNA (final volume of polyplex 200µl) will be shown. Formulation of other polyplexes were performed in a similar manner. The shielding anionic polymer according to the invention amount is calculated as follows: once established the amount of amines required for the NP8 ratio, are divided by 2, half of them will be employed for polymer-DNA interaction and the other half will be confronted with the required shielding anionic polymer NP ratio (NP1).

.. First, the shielding polymer stock solution in water at 10 mg/ml and the polycationic polymer stock solution in water at 4 mg/ml were prepared. The experimental procedure was performed as follows:
1. Add 80µl of PBS in an Eppendorf tube. Then, dilute 20µl of pDNA from a stock of 1mg/ml, and add 15.8µl of the shielding polymer from a stock of 10 mg/ml (final volume of 115.8µl).
2. Add 18.2µl from the polycationic polymer solution (Stock 4 mg/ml) to the pDNA-Shielding polymer solution, and complete with PBS to a final volume of 200µl (66µl PBS).
3. Incubate for 20 minutes at room temperature.
4. The polyplex is ready to be used.

.. Those samples formulated by this first method were prepared in a similar manner for its in-vitro testing. After 24 hours of incubation the toxicity and transfection efficiency were evaluated. The ratios studied for each polymer were N/P 8, 15 or 30. As a positive control for the transfection jetPEI^{®} (Polyplus-transfection S.A, Illkirch, France) (Ref Polyplus: 101-10N) was used at nitrogen to phosporus ratio (NP5). Cell transfection was performed using jetPEI^{®} according to the manufacturer's instructions. jetPEI^{®} is mainly composed of a linear polyethylenimine manufactured at Polyplus-transfection. jetPEI^{®} is provided as a 7.5 mM solution in sterile and apyrogenic water (expressed as concentration of nitrogen residues).

### Example 4. Size and stability of shielded polyplexes

.. Stability of polyplexes is a paramount aspect on developing efficient therapies. Ensuring mid-long term stability of the drug product formulation is followed by a panel assay to mimic the physiological conditions that the drug will meet following the administration route where they need be stable during circulation to the target site of action. It is well known that polyplexes displaying positive surface charges undergo salt-induced agglomeration which might cause inaccurate cell biology evaluation and severe toxicity issues when applied systemically. Initial stability studies are currently under development during the present project, they are aimed to monitor polyplex particle characteristics (size).

.. Size of the stabilized polyplexes formed with pDNA, mRNA or IcDNA at different N/P ratios, different polycations and shielding polymer were performed using a Malvern ZetasizerNanoZS instrument, equipped with a 532 nm laser at a fixed scattering angle of 173. 20µl of the samples were measured using a quartz glass high performance cuvette (Hellma Analytics). Size distribution was measured (diameter, nm) with n > 3 measurements. For the stability measurements, the polyplexes were kept in the fridge (2-8°C) during the experiment and the stability of the polyplexes was measured at different times.

### 4.1. Stabilization of polyplexes formed by N1 and V1 and pDNA as cargo.

.. The stability at different times and the formation of the **N1** polyplexes by polyplex formulation procedure 1 (as reported above), using different NP ratios (8 and 15) and using shielding polymer **V1** different -/+ charge ratio in PBS pH7.4 were studied. For this experiment, different amounts of genetic material were used too (shown in table 3). The final polyplex solution (200µl) was allowed to stabilize for 20 min before measuring the size by DLS (Malvern Panalytical, Spain). The polyplexes were kept in the fridge during the experiment, and the stability of the polyplexes was measured at different times.

.. As shown in table 6, the presence of the shielding polymer provided enhanced stability to the polyplex in solution up to, at least, several days, maintaining a constant size over time and avoiding aggregation.

**Table 6. Stabilization of polyplexes formed by different N/P ratios of N1 and different -/+ charge ratios of V1.**

| Polyplex | Genetic material mass | D(n) / nm t = 0 h | D(n) / nm t = 24 h | D(n) / nm t = 5 days |
|---|---|---|---|---|
| **PX_{NI_8_V1-0}^{pDNA}** | 1µg | N/A | N/A | N/A |
| **PX_{N1_15_V1_1}^{pDNA}** | 1µg | 69,9 ± 3 | 64 ± 3 | 66 ± 1 |
| **PX_{N1_15_V1_0.5}^{pDNA}** | 1µg | 137 ± 6 | 150 ± 5 | 135 ± 4 |
| **PX_{N1_8_V1_1}^{pDNA}** | 1µg | 73 ± 2 | 79 ± 1 | 81 ± 2 |
| **PX_{N1_8_V1_0.5}^{pDNA}** | 1µg | 164 ± 3 | 160 ± 16 | 105 ± 10 |

| | | | | |
|---|---|---|---|---|
| D(n) stands for hydrodynamic diameter measured by DLS; N/A stands for not able to be measured because aggregation was present. | | | | |

.. As can be observed in the table, the size of the polyplexes depends on the mass of genetic material and the ratio of shielding polymer present in the final formulation. Those polyplexes formulated without shielding polymer (i.e. PX_{N1_8_V1_0}^{pDNA} gave big aggregates unable to be measured by the DLS technique.

### 4.2. Stabilization of polyplexes formed by N2 and V1 and pDNA as cargo.

.. As shown in table 7, the presence of the shielding polymer provided enhanced stability to the polyplex formulated by the procedure depicted in example 4.1 in solution up to, at least, several days, maintaining a constant size over time and avoiding aggregation.

**Table 7. Stabilization of polyplexes formed by N2 by different amounts of V1 in different genetic material and different masses of genetic material.**

| **Polyplex** | **Genetic material mass** | **D(n) / nm t = 0h** | **D(n) / nm t = 24h** | **D(n) / nm t = 4days** |
|---|---|---|---|---|
| **PX_{N2_5_V1_0}^{pDNA}** | 1µg | N/A | N/A | N/A |
| **PX**_{**N**2_}**_{5_V1_1}^{pDNA}** | 1µg | 102 ± 12 | 102 ± 13 | 104 ± 15,1 |
| **PX**_{N2_5_}**_{V1_0.5}^{pDNA}** | 1µg | 129 ± 13 | 108 ± 19 | 121 ± 15,5 |

| | | | | |
|---|---|---|---|---|
| D(n) stands for hydrodynamic diameter measured by DLS; N/A stands for not able to be measured because aggregation was present. | | | | |

### 4.3. Stabilization of polyplexes formed by N1 or N3 and V1 and mRNA as cargo.

.. Polyplex stability in PBS pH7.4 using NP=15 with mRNA was studied at different times using the shielding polymer **V1** using +/- =0.5, 1, 2. The final polyplex solution was allowed to stabilize for 20 min before measuring the size by DLS (Malvern Panalytical, Spain). The polyplexes were kept in the fridge during the experiment, and the stability of the polyplexes was measured at different times.

.. The results have demonstrated that **N1** and **N3** complexing polymers at NP ratio 20 are stable with **V1** no matters the +/- ratio employed for polyplex stabilisation during 24 hours employing mRNA as genetic material. It is worth to be mentioned, as the +/- ratio is increased the stability duration is prolonged for at least 5 days. This phenomena is linked with the fact of the raising concentrations of shielding leads to a higher stabilisation characteristics. As shown in table 8, the presence of the shielding polymer promotes the stabilization of the polyplexes.

**Table 8: Stabilization of polyplexes formed by N1 and N3 by different amounts of V1 using mRNA and different times.**

| **Polyplex** | **Genetic material mass** | **D(n) / nm t = 0h** | **D(n) / nm t = 24h** | **D(n) / nm t = 48h** | **D(n) / nm t = 5days** |
|---|---|---|---|---|---|
| **PX**_{**N1**_}**_{15_V1_0.5}^{mRNA}** | 1 µg | 138 ± 13 | 190 ± 1 | 188 ± 6 | 261 ± 1 |
| **PX_{N1_15_V1_1}^{mRNA}** | 1 µg | 130 ± 8 | 127 ± 9 | 124 ± 10 | 135 ± 5 |
| **PX**_{**N1_15_V**1}**_{_2}^{mRNA}** | 1µg | 106 ± 3 | 143 ± 10 | 105 ± 8 | 78 ± 15 |
| **PX_{N3_15_V1_0.5}^{mRNA}** | 1 µg | 143 ± 17 | 148 ± 18 | 7 ± 1 | N/A |
| **PX**_{**N3_15_V1**_}**₁^{mRNA}** | 1 µg | 126 ± 6 | 139 ± 8 | 142 ± 10 | 255 ± 1 |
| **PX_{N3_15_V1_2}^{mRNA}** | 1µg | 140 ± 9 | 141 ± 11 | 143 ± 8 | 167 ± 22 |

| | | | | | |
|---|---|---|---|---|---|
| D(n) stands for hydrodynamic diameter measured by DLS; N/A stands for not able to be measured because aggregation was present. | | | | | |

### 4.4. Stabilization of polyplexes formed by N1 or N3 and V1 and IcDNA as cargo.

.. Polyplex stability in PBS pH7.4 using NP=20 with IcDNA was studied at different times using the shielding polymer **V1** using +/- =0.5, 1, 2. The final polyplex solution was allowed to stabilize for 20 min before measuring the size by Stunner (Unchained Labs, Belgium). The polyplexes were kept in the fridge during the experiment, and the stability of the polyplexes was measured at different times.

.. The use of a different kind of genetic material led to similar results as the previously described in table 9, it is to be noted that **V1** +/- 1 and 2 provided polyplexes stables for at least 7 days. As shown in table 9, the presence of the shielding polymer promotes the stabilization of the polyplexes when IcDNA is employed.

**Table 9: Stabilization of polyplexes formed by N1 and N3 by different amounts of V1 using IcDNA and different times.**

| **Polyplex** | **Genetic material mass** | **Z-ave / nm t = 0h** | **Z-ave / nm t = 7days** |
|---|---|---|---|
| **PX**_{**N1**_**20_V1**_}**_{0.5}^{lcDNA}** | 11.25µg | 258 ± 10 | N/A |
| **PX**_{**N1**_**20_V1_1**} **^{lcDNA}** | 11.25µg | 189 ± 4 | 205 ± 5 |
| **PX_{N1_20_V1_2} ^{lcDNA}** | 11.25µg | 185 ± 3 | 214 ± 3 |
| **PX_{N3_20_V1_0.5} ^{lcDNA}** | 11.25µg | 1114 ± 647 | N/A |
| **PX_{N3_20_V1_1} ^{lCDNA}** | 11.25µg | 216 ± 3 | 206 ± 5 |
| **PX_{N3_20_V1_2} ^{lcDNA}** | 11.25µg | 195 ± 2 | 205 ± 1 |

| | | | |
|---|---|---|---|
| Z-ave stands for hydrodynamic diameter measured by Stunner; N/A stands for not able to be measured because aggregation was present. | | | |

### 4.5. Stabilization of polyplexes formed by N4, N5, N6 or N7 and V1 and pDNA as cargo.

.. Polyplex stability in PBS pH7.4 using NP=15 with pDNA and different complexing polymers was studied at different times using the shielding polymer V1 using +/- =0.5, 1, 2. The final polyplex solution was allowed to stabilize for 20 min before measuring the size by DLS (Malvern Panalytical, Spain). The polyplexes were kept in the fridge during the experiment, and the stability of the polyplexes was measured at different times.

.. As shown in table 10, **V1** can also be employed for stabilisation of polyplexes made of a different chemical nature. These systems were stable for at least 1 day when PBS is employed for its formulation. In addition, some of these polymers **N6** nbu-Poly-L-Ornithine hydrobromide (PLO) and **N7** CHI when they are prepared in acetate buffer provided polyplexes stables for 5 days. The use of acetate buffer is required in order to promote their full protonation of their residues or their limited solubility at a neutral pH. Also, linear **N5** Poly-L-Lysine and **N4** Star-like Poly-L-Lysine have been tested.

**Table 10: Stabilization of polyplexes formed by NX by different amounts of V1 using pDNA and different times.**

| **Polyplex** | **Genetic material mass** | **D(n) / nm t = 0h** | **D(n) / nm t = 24h** | **D(n) / nm t = 48h** | **D(n) / nm t=5days** |
|---|---|---|---|---|---|
| **PX_{N4_15_V1_0.5}^{pDNA}** | 1 µg | 366 ± 190 | 322 ± 32 | N/A | N/A |
| **PX_{N4_15_V1_1}^{Pdna}** | 1 µg | 100 ± 4 | 145 ± 48 | N/A | N/A |
| **PX_{N4_15_V1_2}^{pDNA}** | 1µg | 107 ± 9 | 141 ± 26 | N/A | N/A |
| **PX_{N5_15_V1_0.5}^{pDNA}** | 1 µg | 396 ± 39 | 217 ± 23 | N/A | N/A |
| **PX**_{**N5**_15_**V1**_1}**^{pDNA}** | 1 µg | 115 ± 41 | 158 ± 20 | 116 ± 3 | N/A |
| **PX_{N5_15_V1_2}^{pDNA}** | 1µg | 197 ± 11 | 146 ± 9 | 97 ± 26 | N/A |
| **PX**_{**N6_15_V1**_}**_{0.5}^{pDNA}** | 1µg | 3770 ± 927 | N/A | N/A | N/A |
| **PX_{N6_15_V1_1}^{pDNA}** | 1µg | 159 ± 10 | N/A | N/A | N/A |
| **PX_{N6_15_V2_2}^{pDNA}** | 1µg | 120 ± 2 | N/A | N/A | N/A |
| **PX_{N6_15_V1_0.5}^{pDNA Ac}** | 1µg | 114 ± 20 | 157 ± 13 | 203 ± 44 | N/A |
| **PX_{N6_15_V1_1}^{pDNA Ac}** | 1µg | 136 ± 6 | 174 ± 7 | 178 ± 11 | 241 ± 62 |
| **PX**_{**N6_15_V1**_}**₂^{pDNAAc}** | 1µg | 277 ± 88 | 793 ± 184 | N/A | N/A |
| **PX_{N7_15_V1_0.5}^{pDNA Ac}** | 1µg | 91 ± 24 | 208 ± 92 | 29 ± 12 | N/A |
| **PX_{N7_15_V1_1}^{pDNA Ac}** | 1µg | 72 ± 6 | 126 ± 13 | N/A | N/A |
| **PX**_{**N7**_}**_{15_V1_2}^{pDNA Ac}** | 1µg | 27 ± 1 | 30 ± 7 | N/A | N/A |

| | | | | | |
|---|---|---|---|---|---|
| D(n) stands for hydrodynamic diameter measured by DLS; N/A stands for not able to be measured because aggregation was present. Ac subindex indicate that have been formulated in acetate buffer pH=5 | | | | | |

### Example 5. Complexation/disassembly experiments.

.. In addition, the complexation efficacy and possible presence of free pDNA in the polyplexes were assessed using an electrophoresis gel as a first screening method. To perform the electrophoresis, E-gel Power Snap Electrophoresis Device and E-Gel Power Snap Camera (Invitrogen) was used. 1.2% agarose gels prepared that include the SYBR safe DNA marker (E-Gel^{®} 1.2% with SYBR safe, Invitrogen) were used. The complexation efficiency of the polyplexes (20µl) at different NPs and different -/+ shielding polymer ratio were evaluated, and also the disassembly of the polyplexes in the presence of low heparin (0.075IU/ml) and high heparin (200 IU/ml) concentration (PanReacAppliChem, Spain). For the low concentration, 0.1µl of a 15IU/ml heparin solution was added to the 20µl of already formed polyplex, and for the high concentration, 0.8µl of a 5000IU/ml heparin solution to the 20µl of polyplex was added. Once the gel is loaded (20µl/well), select the equipment protocol according to the type of gel used (in our case, protocol approximately 40min, although the time can be modified according to the samples).

**Table 11. Complexation and disassembly experiments representative of formulated polyplexes synthesized.**

| **Compound** | **Genetic material mass** | **Complexation** | **Disassembly test** |
|---|---|---|---|
| **PX_{N1_8_V1_0}^{pDNA}** | 1 µg | YES | YES (high heparin) |
| **PX_{N1_8_V1_1}^{pDNA}** | 1 µg | YES | NO |
| **PX_{N1_8_V1_1}^{pDNA}** | 1 µg | YES | NO (low heparin) |
| **PX_{N1_8_V1_1}^{pDNA}** | 1 µg | YES | YES (high heparin) |
| **PX_{N1_8_V1_0.5}^{pDNA}** | 1 µg | YES | NO |
| **PX_{N1_8_V1_0.5}^{pDNA}** | 1 µg | YES | NO (low heparin) |
| **PX**_{**N1_8**_}**_{V1_0.5}^{pDNA}** | 1 µg | YES | YES (high heparin) |
| **PX_{N1_15_V1_1}^{pDNA}** | 1 µg | YES | NO |
| **PX_{N1_15_V1_1}^{pDNA}** | 1 µg | YES | NO (low heparin) |
| **PX_{N1_15_V1_1}^{pDNA}** | 1 µg | YES | YES (high heparin) |
| **PX_{N1_15_V1_0.5}^{pDNA}** | 1 µg | YES | NO |
| **PX_{N1_15_V1_0.5}^{pDNA}** | 1 µg | YES | NO (low heparin) |
| **PX_{N1_15_V1_0.5}^{pDNA}** | 1 µg | YES | YES (high heparin) |

.. In all cases, no free pDNA is observed at the different NPs or at low concentrations of heparin. However, at high concentration of heparin, free pDNA signal was observed due to the competition between heparin and pDNA to bind to the polymer, showing the ability of the polymer to release their cargo (Representative image of the gels can be observed in Figure 1).

**Table 12. Complexation experiments representative of formulated polyplexes synthesized.**

| **Compound** | **Genetic material mass** | **Complexation** |
|---|---|---|
| **PX_{N2_8_V1_0}^{pDNA}** | 1 µg | YES |
| **PX_{N2_5_V1_1}^{pDNA}** | 1 µg | YES |
| **PX_{N2_5_V1_0.5}^{pDNA}** | 1 µg | YES |

.. In all cases of PX_{N2_5_V1_1}^{pDNA}, pDNA is disassembled. For PX_{PEI_5_V1_0.5}^{pDNA} is observed free pDNA at high concentrations of heparin. (Representative image of the gels can be observed in Figure 2).

### Example 6A. Cell Culture

.. HeLa cells were cultured in DMEM high glucose with Glutamax (Gibco- Thermo Fisher # 61965-059) supplemented with 10% of Fetal Bovine Serum (Hyclone # SV30160.03HI, provided by GE Healthcare Europe GmbH). Transfections were carried out on 96-well plates containing 10000cells/well in a final volume of 100µl, and cells were incubated 24 hours at 37°C and 5% CO₂. After 24h, the medium was removed and refreshed with 90µl of complete medium. The transfection mixtures were prepared using PBS and in the case of the positive control (JetPEI) manufacturer guidelines were followed (#101-10N, Polyplus Transfection), after 20min of stabilization 10µl of each formulation were added to the cells. After 24 hours cells were recovered and processed.

### Example 6B. ATP Evaluation for Cell Toxicity evaluation

.. After 24h post-incubation, the medium was aspirated and 50µl/well of ATPLite reagent (ATPLite PerkinElmer #6016731) was added. The plate was incubated 10 minutes at room temperature in the dark. Luminiscence was read spectrophotometrically using VictorNivo (PerkinElmer) and data was represented as the percentage of cell viability, taken untreated control cells as 100%.

### Example 6C. Luciferase Assay

.. After 24h post-incubation, 100µl of BrightGlo reagent (Promega # E2620) was added in each well following manufacturer instructions. After 5 minutes of incubation at room temperature luciferase activity was measured using VictorNivo (PerkinElmer). Data was represented as luminescence relative to the percentage of transfection relative to the positive control of transfection.

### Example 6D. Bilogical activity of polyplexes formed by N1 and V1 in HeLa cells

.. The transfection efficiency and the cell viability of the polyplexes formed by N1 and V1 in HeLa cells is reported in the following table. The transfection data is represented as % of the positive control. jetPEI^{®} being the positive control 100% after 24h of treatment and cell viability is compared to non-treated (NT) cells, being the ATP content readout of NT cells equal to 100%.

**Table 13. Cell viability and transfection**

| **Polyplex** | **Genetic material mass** | **Cell viability** | **Transfection** |
|---|---|---|---|
| **PX_{N2_5_V1_0}^{pDNA}** | 1 µg | 67 ± 4 | 100 ± 0 |
| **PX_{N1_8_V1_0}^{pDNA}** | 1 µg | 110 ± 16 | 127 ± 9 |
| **PX**_{**N1**_8**_V1**_}**_{0.5}^{pDNA}** | 1 µg | 90 ± 10 | 253 ± 65 |
| **PX_{N1_8_V1_1}^{pDNA}** | 1 µg | 102 ± 5 | 366 ± 40 |
| **PX_{N1_15_V1_0}^{pDNA}** | 1 µg | 98 ± 6 | 176 ± 10 |
| **PX_{N1_15_V1_0.5}^{pDNA}** | 1 µg | 74 ± 6 | 477 ± 46 |
| **PX_{N1_15_V1_1}^{pDNA}** | 1 µg | 85 ± 22 | 452 ± 10 |

.. As can be extracted from the data described above, the presence of the shielding polymer not only increases the cell viability in HeLa cells, confering to the polymer complex less toxicity but increases the transfection efficiency up to 3 fold.

### Citation List

WO2019067676
J Mol Med, 2002, vol. 80, pp. 648-654

## Claims

1. A polymer complex comprising
a) a positively charged nanoparticle comprising a cationic polymer, a pharmaceutically acceptable salt thereof, or any stereoisomer or mixtures of stereoisomers, either of the compound of cationic polymer, or of any of its pharmaceutically acceptable salts; the cationic polymer being covalently or electrostatically bonded with at least one pharmaceutically, veterinary or cosmetically active ingredient; and
b) at least one anionic copolymer selected from
i. a copolymer comprising substructure I;
ii. a copolymer comprising substructure II; and
iii. a copolymer comprising substructure III;
wherein the substructures I II, and III are depicted as follows:
-Aₘ-Bₙ- Substructure I
-(Bₙ-Aₘ)ₚ-Bₙ- Substructure II
-(Aₘ-Bₙ)ₚ-Aₘ- Substructure III
each instance of A is an amino acid residue is independently selected from methionine sulfoxide, ethionine sulfoxide, S-alkyl-cysteine sulfoxide, S-alkyl cysteine sulfone, S-alkyl homocysteine, S-alkyl homocysteine sulfoxide, glycosylated cysteine, serine, homoserine, homomethionine sulfoxide, sarcosine, glycine, and alanine;
wherein at least 50 mol% of the A amino acid residues are methionine sulfoxide;
wherein each instance of B is independently selected from glutamic acid, aspartic acid, and a salt thereof;
m is an integer from 20 to 600;
n is an integer from 5 to 200;
p is an integer from 1 to 2.

2. The polymer complex according to claim 1, wherein from 60 mol% to 98 mol% of the A amino acid residues are methionine sulfoxide, and the remaining A amino acid residues are selected from sarcosine, glycine and alanine.

3. The polymer complex according to claim 1, wherein 100 mol% of the A amino acid residues are methionine sulfoxide.

4. The polymer complex according to any of claims 1-3, wherein
m is an integer from 25 to 300;
n is an integer from 8 to 150.

5. The polymer complex according to any of claims 1-4, wherein
m is an integer from 25 to 180;
n is an integer from 10 to 100.

6. The polymer complex according to any of claims 1-5, wherein at least one cell-targeting agent; at least one labelling or imaging agent; or at least one cell-targeting agent and at least one labelling or imaging agent is covalently linked to the polypeptidic backbone of the cationic polymer or to the anionic copolymer, through an amino acid side residue; a C or N-terminal group via amide, ester, or anhydride bonding or through a linker.

7. The polymer complex according to any of claims 1-6, wherein the positively charged nanoparticle forms a core portion, and the anionic copolymer forms a shell portion.

8. The polymer complex according to any of claims 1-7, wherein the at least one pharmaceutically, veterinary or cosmetically active ingredient is selected from the group consisting of low molecular weight drugs, peptides, antibodies, nucleic acids, aptamers, and combinations thereof.

9. The polymer complex according to any of claims 1-8, wherein the nucleic acid is selected from the group consisting of DNA/RNA hybrid, a short interfering RNA (siRNA), a microRNA (miRNA), sgRNA, a donorDNA, a self-amplyfing/replicating RNA, a circularRNA (oRNA), a plasmid DNA (pDNA), a linear-closed DNA (IcDNA), a short hairpin RNA (shRNA), messenger RNA (mRNA), and antisense RNA (aRNA), a messenger RNA (mRNA), a CRISPR guide RNA, an antisense nucleic acid, a decoy nucleic acid, an aptamer, and a ribozyme.

10. Use of at least one anionic copolymer selected from
i. a copolymer comprising substructure I;
ii. a copolymer comprising substructure II; and
iii. a copolymer comprising substructure III;
as shielding for positively charged nanoparticles comprising a cationic polymer covalently or electrostatically bonded with at least one pharmaceutically, veterinary or cosmetically active ingredient, for delivery of pharmaceutically, veterinary or cosmetically active ingredients to a biological target;
wherein substructures I, II, and III are as defined in claim 1.

11. A pharmaceutical, veterinary or cosmetical composition comprising at least one polymer complex as defined in any of claims 1-9 together with one or more appropriate acceptable excipients.

12. The polymer complex according to any of claims 1-9, or the composition according to claim 11, for use in a method of delivering at least one pharmaceuticallly, veterinay or cosmetically active ingredient to a biological target, the method comprising:
a) providing the polymer complex according to any of claims 1-9, or the composition of claim 11;
b) contacting the biological target with the polymer complex or the composition.

13. A method for the preparation of a polymer complex which comprises
a) providing a cationic polymer in a first liquid;
b) providing at least one pharmaceuticallly, veterinary or cosmetically active ingredient in a second liquid and mixing it with an anionic copolymer selected from
i. a copolymer comprising substructure I;
ii. a copolymer comprising substructure II; and
iii. a copolymer comprising substructure III;
c) contacting the cationic polymer in the first liquid with the at least one pharmaceuticallly, veterinary or cosmetically active ingredient and the anionic copolymer in the second liquid to form a shielded nanoparticle;
wherein the substructures I, II, and III are as defined herein.

14. The polymer complex according to any of claims 1-9, or the composition according to claim 11, for use as a medicament.

15. The polymer complex according to any of claims 1-9, or the composition according to claim 11, for use (i) as transfection reagent for transfecting at least one active agent into a cell; (ii) for use in the in vivo or ex vivo therapies encoding a peptide or an antibody; iii) in the production of peptides, antibodies, or recombinant virus; (iv) for use as a therapeutic or prophylactic vaccine against viral infections or as a therapeutic vaccine against cancers; and (v) for use in genome engineering, for cell reprogramming, for differentiating cells or for gene-editing.

16. A device for delivering at least one pharmaceutically, veterinary or cosmetically active ingredient into a cell, comprising the polymer complex according to any one of claims 1-9 or the composition according to claim 11.

## Patentansprüche

1. Ein Polymerkomplex, umfassend
a) ein positiv geladenes Nanopartikel, umfassend ein kationisches Polymer, ein pharmazeutisch akzeptables Salz davon oder ein beliebiges Stereoisomer oder Gemische von Stereoisomeren, entweder der Verbindung kationischen Polymers oder eines seiner pharmazeutisch akzeptablen Salze; wobei das kationische Polymer kovalent oder elektrostatisch an mindestens einen pharmazeutischen, tiermedizinischen oder kosmetischen Wirkstoff gebunden ist; und
b) mindestens ein anionisches Copolymer, ausgewählt aus
i. einem Copolymer, das die Unterstruktur I umfasst;
ii. einem Copolymer, das die Unterstruktur II umfasst; und
iii. einem Copolymer, das die Unterstruktur III umfasst;
wobei die Unterstrukturen I, II und III wie folgt dargestellt sind:
-Aₘ-Bₙ- Unterstruktur I
-(Bₙ-Aₘ)ₚ-Bₙ- Unterstruktur II
-(Aₘ-Bₙ)ₚ-Aₘ- Unterstruktur III
jedes Exemplar von A ein Aminosäurerest ist, der unabhängig aus Methioninsulfoxid, Ethioninsulfoxid, S-Alkylcysteinsulfoxid, S-Alkylcysteinsulfon, S-Alkylhomocystein, S-Alkylhomocysteinsulfoxid, glykosyliertem Cystein, Serin, Homoserin, Homomethioninsulfoxid, Sarcosin, Glycin und Alanin ausgewählt ist;
wobei mindestens 50 Mol-% der A-Aminosäurereste Methioninsulfoxid sind;
wobei jedes Exemplar von B unabhängig ausgewählt ist aus Glutaminsäure, Asparaginsäure und einem Salz davon;
m eine ganze Zahl von 20 bis 600 ist;
n eine ganze Zahl von 5 bis 200 ist;
p eine ganze Zahl von 1 bis 2 ist.

2. Der Polymerkomplex nach Anspruch 1, wobei von 60 Mol-% bis 98 Mol-% der A-Aminosäurereste Methioninsulfoxid sind und die verbleibenden A-Aminosäurereste aus Sarcosin, Glycin und Alanin ausgewählt sind.

3. Der Polymerkomplex nach Anspruch 1, wobei 100 Mol-% der A-Aminosäurereste Methioninsulfoxid sind.

4. Der Polymerkomplex nach einem der Ansprüche 1 bis 3, wobei
m eine ganze Zahl von 25 bis 300 ist;
n eine ganze Zahl von 8 bis 150 ist.

5. Der Polymerkomplex nach einem der Ansprüche 1 bis 4, wobei
m eine ganze Zahl von 25 bis 180 ist;
n eine ganze Zahl von 10 bis 100 ist.

6. Der Polymerkomplex nach einem der Ansprüche 1 bis 5, wobei mindestens ein Zellzielmittel; mindestens ein Markierungs- oder Bildgebungsmittel; oder mindestens ein Zellzielmittel und mindestens ein Markierungs- oder Bildgebungsmittel kovalent an das polypeptidische Rückgrat des kationischen Polymers oder an das anionische Copolymer über einen Aminosäureseitenrest; eine C- oder N-terminale Gruppe über Amid-, Ester- oder Anhydridbindung oder über einen Linker gebunden ist.

7. Der Polymerkomplex nach einem der Ansprüche 1 bis 6, wobei das positiv geladene Nanopartikel einen Kernabschnitt bildet und das anionische Copolymer einen Schalenabschnitt bildet.

8. Der Polymerkomplex nach einem der Ansprüche 1 bis 7, wobei der mindestens eine pharmazeutische, tiermedizinische oder kosmetische Wirkstoff aus der Gruppe ausgewählt ist, die aus Arzneimitteln mit niedrigem Molekulargewicht, Peptiden, Antikörpern, Nukleinsäuren, Aptameren und Kombinationen davon besteht.

9. Der Polymerkomplex nach einem der Ansprüche 1 bis 8, wobei die Nukleinsäure ausgewählt ist aus der Gruppe bestehend aus DNA/RNA-Hybrid, einer Smallinterfering-RNA (siRNA), einer microRNA (miRNA), sgRNA, einer Donor-DNA, einer selbstamplifizierenden/replizierenden RNA, einer zirkulären RNA (oRNA), einer Plasmid-DNA (pDNA), einer linear geschlossenen DNA (IcDNA), einer Small hairpin RNA (shRNA), Messenger-RNA (mRNA) und Antisense-RNA (aRNA), einer Messenger-RNA (mRNA), einer CRISPR-Guide-RNA, einer Antisense-Nukleinsäure, einer Köder-Nukleinsäure, einem Aptamer und einem Ribozym.

10. Verwendung von mindestens einem anionischen Copolymer ausgewählt aus
i. einem Copolymer, das die Unterstruktur I umfasst;
ii. einem Copolymer, das die Unterstruktur II umfasst; und
iii. einem Copolymer, das die Unterstruktur III umfasst;
als Abschirmung für positiv geladene Nanopartikel, die ein kationisches Polymer umfassen, das mit mindestens einem pharmazeutischen, tiermedizinischen oder kosmetischen Wirkstoff kovalent oder elektrostatisch verbunden ist, zur Abgabe von pharmazeutischen, tiermedizinischen oder kosmetischen Wirkstoffen an ein biologisches Ziel;
wobei die Unterstrukturen I, II und III wie in Anspruch 1 definiert sind.

11. Eine pharmazeutische, tiermedizinische oder kosmetische Zusammensetzung umfassend mindestens einen Polymerkomplex, wie in einem der Ansprüche 1 bis 9 definiert, zusammen mit einem oder mehreren geeigneten akzeptablen Hilfsstoffen.

12. Der Polymerkomplex nach einem der Ansprüche 1 bis 9 oder die Zusammensetzung nach Anspruch 11 zur Verwendung in einem Verfahren zur Abgabe von mindestens einem pharmazeutischen, tiermedizinischen oder kosmetischen Wirkstoff an ein biologisches Ziel, wobei das Verfahren Folgendes umfasst:
a) bereitstellen des Polymerkomplexes nach einem der Ansprüche 1 bis 9 oder der Zusammensetzung nach Anspruch 11;
b) in-Kontakt-bringen des biologischen Ziels mit dem Polymerkomplex oder der Zusammensetzung.

13. Ein Verfahren zur Herstellung eines Polymerkomplexes, welches Folgendes umfasst
a) bereitstellen eines kationischen Polymers in einer ersten Flüssigkeit;
b) bereitstellen mindestens eines pharmazeutischen, tiermedizinischen oder kosmetischen Wirkstoffs in einer zweiten Flüssigkeit und Mischen dessen mit einem anionischen Copolymer, ausgewählt aus
i. einem Copolymer, das die Unterstruktur I umfasst;
ii. einem Copolymer, das die Unterstruktur II umfasst; und
iii. einem Copolymer, das die Unterstruktur III umfasst;
c) in-Kontakt-bringen des kationischen Polymers in der ersten Flüssigkeit mit dem mindestens einen pharmazeutischen, tiermedizinischen oder kosmetischen Wirkstoff und dem anionischen Copolymer in der zweiten Flüssigkeit, um ein abgeschirmtes Nanopartikel zu bilden;
wobei die Unterstrukturen I, II und III wie hierin definiert sind.

14. Der Polymerkomplex nach einem der Ansprüche 1 bis 9 oder die Zusammensetzung nach Anspruch 11 zur Verwendung als Medikament.

15. Der Polymerkomplex nach einem der Ansprüche 1 bis 9 oder die Zusammensetzung nach Anspruch 11 zur Verwendung (i) als Transfektionsreagenz zur Transfektion von mindestens einem Wirkstoff in eine Zelle; (ii) zur Verwendung in den in vivo oder ex vivo Therapien, die ein Peptid oder einen Antikörper kodieren; (iii) bei der Herstellung von Peptiden, Antikörpern oder rekombinantem Viren; (iv) zur Verwendung als therapeutischer oder prophylaktischer Impfstoff gegen virale Infektionen oder als therapeutischer Impfstoff gegen Krebs; und (v) zur Verwendung in der Genomtechnik, zur Zellreprogrammierung, zur Differenzierung von Zellen oder zur Genome Editing.

16. Eine Vorrichtung zur Abgabe von mindestens einem pharmazeutischen, tiermedizinischen oder kosmetischen Wirkstoff in eine Zelle, die den Polymerkomplex nach einem der Ansprüche 1 bis 9 oder die Zusammensetzung nach Anspruch 11 umfasst.

## Revendications

1. Un complexe polymère comprenant
a) une nanoparticule chargée positivement comprenant un polymère cationique, un sel pharmaceutiquement acceptable de celui-ci, ou tout stéréoisomère ou mélange de stéréoisomères, soit du composé de polymère cationique, soit de l'un de ses sels pharmaceutiquement acceptables ; le polymère cationique étant lié de manière covalente ou électrostatique à au moins un ingrédient pharmaceutiquement, vétérinairement ou cosmétiquement actif ; et
b) au moins un copolymère anionique choisi parmi
i. un copolymère comprenant la sous-structure I ;
ii. un copolymère comprenant la sous-structure II ; et
iii. un copolymère comprenant la sous-structure III ;
dans lequel les sous-structures I, II et III sont représentées comme suit :
-Aₘ-Bₙ- Sous-structure I
-(Bₙ-Aₘ)ₚ-Bₙ- Sous-structure II
-(Aₘ-Bₙ)ₚ-Aₘ- Sous-structure III
chaque instance de A est un résidu d'acide aminé indépendamment choisi parmi le sulfoxyde de méthionine, le sulfoxyde d'éthionine, le sulfoxyde de S-alkyl-cystéine, la sulfone de S-alkyl-cystéine, la S-alkyl homocystéine, le sulfoxyde de S-alkyl homocystéine, la cystéine glycosylée, la sérine, l'homosérine, le sulfoxyde d'homométhionine, la sarcosine, la glycine et l'alanine ;
dans lequel au moins 50 % en moles des résidus d'acides aminés A sont du sulfoxyde de méthionine ;
dans lequel chaque instance de B est indépendamment choisie parmi l'acide glutamique, l'acide aspartique et un sel de ceux-ci ;
m est un nombre entier allant de 20 à 600;
n est un nombre entier allant de 5 à 200 ;
p est un nombre entier allant de 1 à 2.

2. Le complexe polymère selon la revendication 1, dans lequel de 60 % en moles à 98 % en moles des résidus d'acides aminés A sont de méthionine sulfoxyde, et les résidus d'acides aminés A restants sont choisis parmi la sarcosine, la glycine et l'alanine.

3. Le complexe polymère selon la revendication 1, dans lequel 100 % en moles des résidus d'acides aminés A sont du sulfoxyde de méthionine.

4. Le complexe polymère selon l'une quelconque des revendications 1 à 3, dans lequel
m est un nombre entier allant de 25 à 300;
n est un nombre entier allant de 8 à 150.

5. Le complexe polymère selon l'une quelconque des revendications 1 à 4, dans lequel
m est un nombre entier allant de 25 à 180;
n est un nombre entier allant de 10 à 100.

6. Le complexe polymère selon l'une quelconque des revendications 1 à 5, dans lequel au moins un agent de ciblage cellulaire ; au moins un agent de marquage ou d'imagerie ; ou au moins un agent de ciblage cellulaire et au moins un agent de marquage ou d'imagerie est lié de manière covalente au squelette polypeptidique du polymère cationique ou au copolymère anionique, par l'intermédiaire d'un résidu latéral d'acide aminé ; un groupe C ou N-terminal par l'intermédiaire d'une liaison amide, ester ou anhydride ou par l'intermédiaire d'un lieur.

7. Le complexe polymère selon l'une quelconque des revendications 1 à 6, dans lequel la nanoparticule chargée positivement forme une partie de noyau, et le copolymère anionique forme une partie d'enveloppe.

8. Le complexe polymère selon l'une quelconque des revendications 1 à 7, dans lequel le au moins un ingrédient pharmaceutiquement, vétérinairement ou cosmétiquement actif est choisi dans le groupe constitué des médicaments à faible masse moléculaire, des peptides, des anticorps, des acides nucléiques, des aptamères et des combinaisons de ceux-ci.

9. Le complexe polymère selon l'une quelconque des revendications 1 à 8, dans lequel l'acide nucléique est choisi dans le groupe constitué par un hybride ADN/ARN, un petit ARN interférent (pARNi), un micro-ARN (miARN), un sgARN, un ADN donneur, un ARN à auto-amplification/réplication, un ARN circulaire (circARN), un ADN plasmidique (pADN), un ADN à fermeture linéaire (IcADN), un petit ARN en épingle à cheveux (shARN), un ARN messager (ARNm) et un ARN antisens (ARNa), un ARN messager (ARNm), un ARN guide CRISPR, un acide nucléique antisens, un acide nucléique leurre, un aptamère et un ribozyme.

10. Utilisation d'au moins un copolymère anionique choisi parmi
i. un copolymère comprenant la sous-structure I ;
ii. un copolymère comprenant la sous-structure II ; et
iii. un copolymère comprenant la sous-structure III ;
comme blindage pour des nanoparticules chargées positivement comprenant un polymère cationique lié de manière covalente ou électrostatique à au moins un ingrédient pharmaceutiquement, vétérinairement ou cosmétiquement actif, pour l'administration d'ingrédients pharmaceutiquement, vétérinairement ou cosmétiquement actifs à une cible biologique ;
dans laquelle les sous-structures I, Il et III sont telles que définies dans la revendication 1.

11. Une composition pharmaceutique, vétérinairement ou cosmétique comprenant au moins un complexe polymère tel que défini dans l'une quelconque des revendications 1 à 9 avec un ou plusieurs excipients acceptables appropriés.

12. Le complexe polymère selon l'une quelconque des revendications 1 à 9, ou la composition selon la revendication 11, pour une utilisation dans un procédé d'administration d'au moins un ingrédient pharmaceutiquement, vétérinairement ou cosmétiquement actif à une cible biologique, le procédé comprenant :
a) fournir le complexe polymère selon l'une quelconque des revendications 1 à 9, ou la composition de la revendication 11 ;
b) mettre en contact la cible biologique avec le complexe polymère ou la composition.

13. Un procédé pour la préparation d'un complexe polymère qui comprend
a) fournir un polymère cationique dans un premier liquide ;
b) fournir au moins un ingrédient pharmaceutiquement, vétérinairement ou cosmétiquement actif dans un second liquide et le mélanger avec un copolymère anionique choisi parmi
i. un copolymère comprenant la sous-structure I ;
ii. un copolymère comprenant la sous-structure II ; et
iii. un copolymère comprenant la sous-structure III ;
c) mettre en contact le polymère cationique dans le premier liquide avec le au moins un ingrédient pharmaceutiquement, vétérinairement ou cosmétiquement actif et le copolymère anionique dans le second liquide pour former une nanoparticule blindée ;
dans lequel les sous-structures I, II et III sont telles que définies ici.

14. Le complexe polymère selon l'une quelconque des revendications 1 à 9, ou la composition selon la revendication 11, pour une utilisation en tant que médicament.

15. Le complexe polymère selon l'une quelconque des revendications 1 à 9, ou la composition selon la revendication 11, pour l'utilisation (i) en tant que réactif de transfection pour transfecter au moins un agent actif dans une cellule ; (ii) pour l'utilisation dans les thérapies in vivo ou ex vivo codant pour un peptide ou un anticorps ; iii) dans la production de peptides, d'anticorps ou de virus recombinants ; (iv) pour l'utilisation en tant que vaccin thérapeutique ou prophylactique contre les infections virales ou en tant que vaccin thérapeutique contre les cancers ; et (v) pour l'utilisation dans l'édition génomique, pour la reprogrammation cellulaire, pour la différenciation des cellules ou pour modification localisée de séquence génomique.

16. Un dispositif pour administrer au moins un ingrédient pharmaceutiquement, vétérinairement ou cosmétiquement actif dans une cellule, comprenant le complexe polymère selon l'une quelconque des revendications 1 à 9 ou la composition selon la revendication 11.
